(19) Europäisches Patentamt
European Patent Office
Office européen des brevets

(11) EP 3 717 017 B1

(12) **EUROPEAN PATENT SPECIFICATION**

(45) Date of publication and mention of the grant of the patent:
**25.02.2026 Bulletin 2026/09**

(21) Application number: **18821915.8**

(22) Date of filing: **03.12.2018**

(51) International Patent Classification (IPC):
***A61K 47/60*** *(2017.01)* ***A61K 47/10*** *(2017.01)*
***A61P 23/02*** *(2006.01)* ***A61P 29/02*** *(2006.01)*
***A61K 9/00*** *(2006.01)*

(52) Cooperative Patent Classification (CPC):
**A61K 47/60; A61K 9/0019; A61K 47/10; A61P 23/02**

(86) International application number:
**PCT/US2018/063573**

(87) International publication number:
**WO 2019/109065 (06.06.2019 Gazette 2019/23)**

(54) **COVALENT ANESTHETIC-POLYMER CONJUGATES FOR PROLONGED LOCAL ANESTHESIA**

POLYMERKONJUGATE MIT KOVALENTEM ANÄSTHETIKUM ZUR VERLÄNGERTEN LOKALANÄSTHESIE

CONJUGUÉS COVALENTS ANESTHÉSIQUES-POLYMÈRES POUR ANESTHÉSIE LOCALE PROLONGÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.12.2017 US 201762593784 P**

(43) Date of publication of application:
**07.10.2020 Bulletin 2020/41**

(73) Proprietor: **The Children's Medical Center Corporation**
**Boston, Massachusetts 02115 (US)**

(72) Inventors:
- **KOHANE, Daniel S.**
  **Newton**
  **Massachusetts 02461 (US)**
- **ZHAO, Chao**
  **Tuscaloosa**
  **Alabama 35406 (US)**

(74) Representative: **Hoffmann Eitle**
**Patent- und Rechtsanwälte PartmbB**
**Arabellastraße 30**
**81925 München (DE)**

(56) References cited:
**EP-A1- 3 315 126** **WO-A1-2016/206549**
**WO-A2-2004/014350**

- **DIEGO A. GIANOLIO ET AL: "Synthesis and Evaluation of Hydrolyzable Hyaluronan-Tethered Bupivacaine Delivery Systems", BIOCONJUGATE CHEMISTRY, vol. 16, no. 6, 1 November 2005 (2005-11-01), US, pages 1512 - 1518, XP055561414, ISSN: 1043-1802, DOI: 10.1021/bc050239a**
- **KOHANE D S ET AL: "Prolonged duration local anesthesia from tetrodotoxin-enhanced local anesthetic microspheres", PAIN, ELSEVIER SCIENCE PUBLISHERS, AMSTERDAM, NL, vol. 104, no. 1, 1 July 2003 (2003-07-01), pages 415 - 421, XP002584889, ISSN: 0304-3959, DOI: 10.1016/S0304-3959(03)00049-6**
- **WEINIGER C F ET AL: "Review of prolonged local anesthetic action", EXPERT OPINION ON DRUG DELI, INFORMA HEALTHCARE, GB, vol. 7, no. 6, 1 June 2010 (2010-06-01), pages 737 - 752, XP009170482, ISSN: 1742-5247, DOI: 10.1517/17425241003767383**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention is in the field of prolonged nerve blocks and local anesthesia and analgesia with decreased toxicity, and more specifically concerns a covalent anesthetic-polymer conjugate for prolonged duration local anesthesia, a formulation comprising the conjugate, a dosage unit comprising the formulation, the dosage unit or formulation for use in a method of providing a nerve blockade, and a method of making the conjugate.

### BACKGROUND OF THE INVENTION

Prolonged duration local anesthesia following a single injection has been a focus of clinical and scientific research in the last two decades. However, there are many limitations associated with the conventional amino-ester and amino-amide local anesthetic compounds currently used in the clinic. Even though these compounds cause effective peripheral nerve blockade, their relatively short duration of action is often inadequate, especially in the management of chronic and neuropathic pain. These compounds also cause side effects, such as local toxicity to the muscle and the peripheral nerves, which increase with higher concentrations and longer durations of exposure. Unfortunately, these side effects are worsened when vehicles of sustained release are used to deliver these compounds (even though the delivery vehicle themselves are minimally toxic) and can cause inflammatory responses at the nerve which sometimes considerably outlast the duration of nerve blockade (Padera, et al. Anesthesiology 108, 921-928, doi:10.1097/ALN.0b013e31816c8a48 (2008); Kohane, et al. Pain 104, 415-421, doi:http://dx.doi.org/10.1016/S0304-3959(03)00049-6 (2003); Kohane, et al. Journal of Biomedical Materials Research 59, 450-459, doi:10.1002/jbm.1261 (2002)). In particular, low specificity of many reagents for peripheral nerve voltage-gated sodium channels may cause severe systemic side effects, which are primarily cardiovascular (*e.g.*, life threatening arrhythmias), and neurologic (*e.g.*, seizures).

In light of these limitations, it has been a long-standing goal to develop a local anesthetic formulation that can enhance or prolong nerve blockade with minimal systemic and local side effects. Tetrodotoxin (TTX), a naturally occurring site 1 sodium-channel blocker (S1SCB), has been studied in the last two decades as an alternative to conventional local anesthetics (Lahaye, et al. Anesthesiology 123, 741-742 (2015)). Even though TTX has extremely potent local anesthetic properties (Kohane, et al. Regional Anesthesia and Pain Medicine 25, 52-59, doi:http://dx.doi.org/10.1016/S1098-7339(00)80011-5 (2000)), the doses of TTX required to achieve significant peripheral nerve blockade can cause hypotension and systemic muscular weakness, including diaphragmatic paralysis and respiratory failure. Despite the narrow therapeutic window of TTX, efforts are currently underway to introduce it into clinical practice (Lahaye, et al. Anesthesiology 123, 741-742 (2015); PERE, et al. Regional Anesthesia and Pain Medicine 18, 304-307 (1993); Lobo, et al. Anesthesiology 123, 873-885 (2015)), and an approach for prolonging the nerve blockade duration of TTX for clinical use is desired.

While the encapsulation of drugs into sustained delivery systems can achieve prolonged drug duration, TTX is very hydrophilic, which hinders its effective encapsulation (Rwei, et al. Proceedings of the National Academy of Sciences 112, 15719-15724, doi:10.1073/pnas. 1518791112 (2015); Shankarappa, et al. Proceedings of the National Academy of Sciences 109, 17555-17560, doi:10.1073/pnas.1214634109 (2012)). Liposomes have previously been reported as efficient carriers for the controlled release of TTX with encapsulation efficiencies up to 50 % (Rwei, et al. Proceedings of the National Academy of Sciences 112, 15719-15724, doi:10.1073/pnas.1518791112 (2015); Epstein-Barash, H. et al. Proceedings of the National Academy of Sciences 106, 7125-7130, doi:10.1073/pnas.0900598106 (2009); Zhan, C. et al. Nano Letters 16, 177-181, doi:10.1021/acs.nanolett. 503440 (2016)). These liposomes achieved peripheral nerve blockade that lasted up to 13.5 h in a rat sciatic nerve model, with minimal local or systemic toxicity. However, the initial burst release of TTX prevented further increases in the injected dose and thereby prevented further prolongation of nerve blockade. Ideally, injectable anesthetics for management of postoperative or chronic pain would from several last days up to weeks. Furthermore, time-consuming and cumbersome preparation protocols make liposomal formulations inconvenient for clinical use.

It is therefore an object of the present invention to provide biocompatible platforms for the delivery of anesthetic agents over a prolonged period of time following a single administration, with reduced toxicity.

It is also an object of the present invention to provide biocompatible formulations of specific site one sodium channel blockers (S1SCBs) that prolong the duration of anesthesia from these toxins with reduced local or systemic toxicity.

### SUMMARY OF THE INVENTION

The present invention is defined in the claims.

An aspect of the invention is a covalent anesthetic-polymer conjugate for prolonged duration local anesthesia

comprising:

(a) an anesthetic agent; and
(b) a polymer backbone;

wherein

the polymer backbone comprises one or more hydrophilic or hydrophobic polymers, and optionally one or more polyethylene oxide polymers;
the polymer backbone comprises a polymer produced from a dicarboxylic acid and a triol;
the anesthetic agent is selected from tetrodotoxin (TTX), saxitoxin (STX), dicarbamoyl saxitoxin, neosaxitoxin, and the gonyautoxins;
the anesthetic agent is covalently conjugated to the polymer backbone *via* a hydrolysable linker; and
the anesthetic agent is present in an amount effective to induce effective local nerve blockade with reduced toxicity relative to the unconjugated anesthetic agent following administration to a subject at a site in need thereof.

Conjugates including anesthetic agents, especially site I sodium channel blockers such as tetrodotoxin, covalently bound to a biodegradable and biocompatible polymer backbone *via* a hydrolysable ester linkage provide prolonged local anesthesia with significantly decreased toxicity. The covalent bonds are sufficiently stable to prevent an initial "burst release" of the anesthetic agent, and the slow hydrolysis of ester bonds achieves sustained release of an effective amount of anesthetic to block pain *in vivo* for up to 72 hours, or for more than 72 hours, up to one month, following a single administration. The anesthetic is released in its native form. The hydrolysis rate of ester bonds within the conjugate is tailored according to the needs of the recipient by altering the hydrophilicity of the polymer backbone.

It has also been established that PEG functions as a chemical permeation enhancer that increases the efficacy of the anesthetic within conjugates. Therefore, formulations of anesthetic-polymer conjugates homogeneously dispersed into PEG200 are described. In preferred embodiments, formulations of anesthetic-polymer conjugates are dispersed in PEG200 as a syringe-injectable formulation. Methods of making covalent anesthetic-polymer formulations and the formulations for use in a method for providing a nerve blockade are also described.

The anesthetic agent in the conjugate of the invention is a site one sodium channel blocker (S1SCB) selected from tetrodotoxin (TTX), saxitoxin (STX), decarbamoyl saxitoxin, neosaxitoxin, and the gonyautoxins. In an exemplary embodiment, the S1SCB is tetrodotoxin (TTX).

The conjugates include anesthetic agents covalently bound to biodegradable biocompatible polymers. The hydrophilicity of the polymer can be modified in a controlled manner, for example, by the addition of hydrophilic polymers. Therefore, the conjugates enable the tunable release of anesthetic agents *in vivo* by changing the concentration and relative ratios of the hydrophilic and hydrophobic polymers and monomers in the conjugate. Preferred biocompatible polymer backbones include polyanhydrides. An exemplary polyanhydride polymer is poly(glycerol sebacate) (PGS). Preferred hydrophilic polymers include polyethylene oxide polymers and copolymers, such as poly(ethylene glycol) (PEG). Exemplary PEGs include PEG100, PEG200, PEG300, PEG400, PEG500, PEG600, PEG700, PEG800, PEG900, PEG1000, and PEG2000.

One skilled in the art could understand that the amount of anesthetic agent within the formulations can be tailored according to the needs of the recipient. In some embodiments, the formulations include anesthetic agents in dosages of between 0. 1 $\mu$g and 200 $\mu$g, inclusive, preferably between 1 $\mu$g and 100 $\mu$g, inclusive, per dose.

The duration of effective nerve block resulting from administering formulations of conjugates in vivo can be adjusted by varying the amount and ratio of the hydrophilic polymers in the conjugates. Therefore, in some embodiments, the amount and ratio of the hydrophilic polymers within the conjugate is sufficient to provide effective nerve block over a period of time from one hour, up to one month, according to the needs of the recipient. For example, in some embodiments, the formulations deliver an amount of anesthetic to provide effective nerve blockade for up to 72 hours in in vivo. In other embodiments, the formulations deliver an amount of anesthetic to provide effective nerve blockade for at least 72 hours, up to two weeks, or more than two weeks, for example, up to one month in vivo. The formulations provide effective nerve blockade with minimal systemic toxicity and virtually no local toxicity to the muscle, or the peripheral nerves. In some embodiments, the conjugates include one or more additional therapeutic, prophylactic or diagnostic agents covalently linked to the amphiphilic polymer. In other embodiments, the conjugates are formulated into liposomes or other secondary delivery vehicles, optionally including one or more additional therapeutic, prophylactic or diagnostic agents. In other embodiments, one or more additional therapeutic, prophylactic or diagnostic agents are included for injection with the conjugates by admixture into the injectate. The additional active agents can be covalently linked to the polymer backbone *via* a hydrolysable ester linkage. In some embodiments the additional active agent is a glucocorticoid, such as dexamethasone.

The conjugates of the invention can provide effective nerve blockade for up to one month in the absence of local

toxicity or with significantly reduced local toxicity following a single administration. In some embodiments, a formulation includes an anesthetic agent covalently conjugated onto PGS, optionally modified by the addition of PEG, in an effective amount to provide a nerve block at the site of administration for up to 3 days, up to one month following administration. In some embodiments, the formulation is effective to treat or prevent neuropathic pain in the subject.

## BRIEF DESCRIPTION OF THE DRAWINGS

Figures 1A and 1B are schematic representations of the step-wise synthesis of a covalent conjugate of an anesthetic (tetrodotoxin; TTX) and a di-carboxylic acid/Triol polymer (TD) to form a TD-TXX conjugate (Figure 1A), or with the inclusion of PEG (P) to form a TDP-TXX conjugate (Figure 1B), respectively. Figure 1C is a schematic representation of the molecular model of a PGS-PEG-TTX conjugate "lattice". TTX is represented as (•), and PGS-PEG is represented as (-).

Figures 2A-2C are line graphs, showing contact angle (degrees) over $f_{phil}$ (%) (Figure 2A); $T_{1/2}$ Mass loss (h) over $f_{phil}$ (%) (Figure 2B); and Mass loss (%) over Time (hours) for each of TgD8 (-■-); TgD8P200 (-•-); TgD8P1000 (-▲-); and TgD8P2000 (-▼-) (Figure 2C), respectively.

Figures 3A and 3B are bar graphs showing % cell viability for C2C12 cells (Figure 3A), or PC12 cells (Figure 3B), incubated for 24 hours with each of TgD8; TgD8P200; TgD8P1000; and TgD8P2000, each at dosages of 0.001 mg/ml (left bar), 0.01 mg/ml (middle bar) and 0.1 mg/ml (right bar), respectively. Data are means ± SD, n=4.

Figures 4A-4B are line graphs, showing $T_{1/2}$ Mass loss (h) over $f_{phil}$ (%) (Figure 4A), and cumulative TTX release (%) over time (0-800 hours), for each of TgD8 (-■-); TgD8P200 (-•-); TgD8P1000 (-▲-); and TgD8P2000 (-▼-); and free TTX (-♦-), respectively (Figure 4B).

Figures 5A and 5B are schematic representations of the step-wise synthesis of a covalent conjugate of an anesthetic (tetrodotoxin; TTX) and a di-carboxylic acid/Triol polymer (TD), either with the inclusion of one or more additional "linking" polymers to form a TD Polymer-TXX conjugate (Figure 5A), or including Dexamethasone (Dex) to form a TD-Dex-TXX conjugate (Figure 5B), respectively.

Figures 6A-6E are line graphs. Figure 6A shows cumulative Dex release (%) over Time (hours) for each of TgD8 (-■-); TgD8P200 (-•-); TgD8P1000 (-▲-); and TgD8P2000 (-▼-); and free TTX (-♦-), respectively. Figures 6B-6E show cumulative drug release (%), and Mass loss (%) from 25 mg each PGS-PEG-drug conjugate, over time (h), for each of PGS-PEG2000-TTX/dexamethasone. (Figure 6B); PGS-PEG1000-TTX/dexamethasone (Figure 6C); PGS-PEG200-TTX/dexamethasone (Figure 6D); and PGS-TTX/dexamethasone (Figure 6E), respectively. TTX is represented as (-•-), dexamethasone (Dex) is represented as (-▲-), and Mass Loss is represented as (■).

Figure 7 is a schematic representation of the solvent evaporation process to prepare an injectable formulation of a PGS-PEG-TTX conjugate in PEG 200.

Figures 8A-8C are line graphs. Figure 8A shows Complex viscosity (Pa.s) over $f_{phil}$ (%) for each of 0.1 rad/s (■); 1 rad/s (•); and 10 rad/s (A), respectively. Figure 8B shows the Complex Viscosity (Pa.s) over Angular frequency (rad/s) for each of PGS-TTX in PEG200 (■); of PGS-PEG200-TTX in PEG200 (•); of PGS-PEG1000-TTX in PEG200 (A); of PGS-PEG2000-TTX in PEG200 (▼); and PEG200 (♦), respectively. Figure 8C shows the Modulus G', G" (Pa) over Angular frequency (rad/s) for each of G"(■), and G' (•), respectively.

Figures 9A-9D are line graphs showing % Successful blocks over TTX dose (μg) (Figure 9A); Duration of block (hours) over TTX dose (Figure 9B); % Contralateral Block over TTX dose (μg) (Figure 9C); and % Mortality over TTX dose (μg) (Figure 9D), respectively, for each of (■) conjugated TTX/PEG200; Free TTX/PBS(•); Free TTX/PEG200 (A), respectively.

Figures 10A to 10E are graphs. Figure 10A is a line graph showing thermal latency (s) over Time (h) for 10 μg TTX in 25 mg of TgD8TTX conjugate in 35 mg TgD8-TTX for each of injected leg (■), and contralateral leg (-•-), respectively. Figure 10B is a line graph showing thermal latency (s) over Time (hours) for 9.2 μg TTX conjugate in 30 mg TgD8P200-TTX for each of injected leg (■), and contralateral leg (-•-), respectively. Figure 10C is a line graph showing thermal latency (s) over Time (hours) for 7 μg TTX in 25 mg of TTX conjugate in 25 mg TgD8P1000 for each of injected leg (■), and contralateral leg (-•-), respectively. Figure 10D is a line graph showing thermal latency (s) over Time (hours) for 4.8 μg TTX in 30 mg of TTX conjugate in 25 mg TgD8P2000 for each of injected leg (■), and contralateral leg (-•-), respectively. Figure 10E is a line graph showing thermal latency (s) over Time (hours) for 4.0 μg of Free TTX for each of injected leg (■), and contralateral leg (-•-), respectively.

Figure 11 is a line graph showing the dose response curves for Duration of blockade in hours over TTX dose (μg) for each of conjugated TTX/PEG200 (■), Free TTX/PBS (-•-), and Free TTX/PEG200 (▲), respectively.

Figures 12A-12D are line graphs. Figures 12A and 12B show %Mortality (Figure 12A) and Duration of Blocks (hours) (Figure 12B) over $f_{phil}$ (%), respectively, for 10 μg TTX conjugate. Figures 12C and 12 D show % Successful blocks (Figure 12C) and Duration of Blocks (hours) (Figure 12D) over $f_{phil}$ (%), respectively, for 1 μg TTX conjugate.

Figure 13 is a line graph showing Relative fluorescence (%) over time post-injection (hours).

Figure 14 is a line graph showing Release of capsaicin from PGS-capsaicin conjugates, as represented by amount of Capsaicin (μg) over time (days) for each of PGS-Cap(■); PGS-Cap-2(-•-); PGS-PEG-1000-Cap(▲); and PGS-PEG-1000-Cap-2(▼), respectively.

## DETAILED DESCRIPTION OF THE INVENTION

### I. Definitions

The term "site 1 sodium channel blocker" or "S1SCB" refers to a molecule that binds the outer opening of sodium channels at a location termed "site 1". In a preferred embodiment, the site 1 sodium channel blocker is a naturally occurring toxin or a derivative thereof. The term "derivative thereof" includes any derivative of a site 1 sodium channel blocker having substantially the same functional properties as the non-derivatized site 1 sodium channel blocker such as biological and/or pharmacological, *i.e.* to effectively block sodium channels.

The term "anesthesia" refers to a loss of sensation (local; not causing loss of consciousness; systemic, with loss of consciousness) and usually of consciousness without loss of vital functions. The terms "anesthetic" and "anesthetic agents" refer to agents that induce anesthesia in a subject.

The term "vasoconstrictor" is an agent causing narrowing of the lumen of blood vessels especially as a result of vasomotor action.

The term "infiltration" refers to injection into multiple layers or areas of tissue.

The term "injection" refers to injection into a single point in tissue or lumen.

The term "nerve block" refers to local anesthesia produced by interruption of the flow of impulses along a nerve trunk.

The term "Minimum effective concentration" (MEC) is the lowest local concentration of one or more drugs in a given location sufficient to provide pain relief.

The terms "individual," "individual," "subject," and "patient" are used interchangeably, and refer to a mammal, including, but not limited to, humans, rodents, such as mice and rats, and other laboratory animals.

The term "biocompatible" refers to one or more materials that are neither themselves toxic to the host (*e.g.,* an animal or human), nor degrade (if the polymer degrades) at a rate that produces monomeric or oligomeric subunits or other byproducts at toxic concentrations in the host.

The term "backbone polymer", or "backbone" refers to the chemical moiety or polymer included within covalent anesthetic-polymer conjugate to which the anesthetic is covalently attached. The backbone polymer does not form part of the excipient. A preferred backbone polymer is poly(glycerol sebacate)(PGS)-PEG copolymer (PGS-PEG).

The term "hydrolysable linker" refers to any chemical bond or moiety that can be degraded hydrolytically, such as an ester bond.

The term "burst release" refers to rapid or release or delivery of an agent, for example, the rapid delivery of an active agent from a delivery vehicle or structure. For example, the rapid or "burst" release of a drug from a drug delivery system *in vivo* may be attributed to the fraction of the drug which is adsorbed or weakly bound to the delivery system, as compared to the slower release of drugs by diffusion or degradation of the delivery system.

The term "pharmaceutically acceptable carrier" includes any and all solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the active compound, use thereof in the therapeutic formulations is contemplated. Supplementary active compounds can also be incorporated into the formulations.

References herein to methods of therapeutic treatment using a conjugate, formulation or dosage form should be understood as directed to the conjugate, formulation or dosage form for use in the method.

### II. Compositions

It has been established that formulations including local anesthetics covalently conjugated onto biodegradable biocompatible polymers such as PGS and PGS-PEG backbones provide tunable release of the anesthetic *in vivo.* The formulations enable local delivery of high concentrations of anesthetics with decreased systemic and local toxicity. The rate and amount of anesthetic released at the site of administration is controllable by varying amount of anesthetic, the molecular weight, composition, and concentration of the polymer(s), as well as the type of covalent linker used to conjugate the molecules.

The anesthetic agents are conjugated to at least one local amphiphilic polymer. Exemplary amphiphilic polymers include poly(glycerol sebacate) (PGS). In some embodiments the PGS conjugates include one or more hydrophilic polymer(s). Exemplary hydrophilic polymers include polyethylene glycol (PEG). Each of these components is discussed in

detail, below. Formulations of covalent anesthetic-polymer conjugates can also include additional active agents, such as therapeutic or diagnostic agents, as well as excipients and preservatives.

### A. Anesthetic Agents

Anesthetic agents are included in the covalent anesthetic-polymer conjugates. Prior to conjugation, the anesthetic agent includes one or more free hydroxyl or carboxyl groups. In some embodiments, the anesthetic agent is modified to include one or more free hydroxyl or carboxyl groups prior to conjugation. A preferred anesthetic agent is Tetrodotoxin (TTX).

#### 1. Site 1 Sodium Channel Blockers (S1SCB)

Site 1 blockers are a family of molecules long recognized for their potent and specific blockade of voltage gated sodium channels. Site I sodium channel blockers (S1SCBs) include phycotoxins (saxitoxin (STX), decarbamoyl saxitoxin, neosaxitoxin (Neo), and the gonyautoxins), tetrodotoxin (TTX), and several of the conotoxins.

#### i. Tetrodotoxin (TTX)

Tetrodotoxin (TTX) is a highly potent neurotoxin that blocks the fast Na+ current in human myocytes (the contractile cells of the muscles), thereby inhibiting their contraction. Chemically, it is an amino polhydroquinoline (see Pharmacological Reviews, Vol. 18 No. 2, pp. 997-1049).

Tetrodotoxin alone is too toxic to be used as an anesthetic. Combinations of tetrodotoxin with bupivacaine produced long duration sciatic nerve blockade in rats without increased systemic toxicity compared to tetrodotoxin alone (Kohane, et al., Anesthesiology, 1998:119-131). Although the potent inhibition of voltage-gated Na+ channels is too hazardous for TTX to be used as a drug alone, blocking such channels in a controlled fashion maybe desirable in the treatment of conditions such as Parkinson's disease and chronic pain in terminally ill cancer patients. Formation of a conjugate turns this highly toxic toxin into a useful and safe anesthetic.

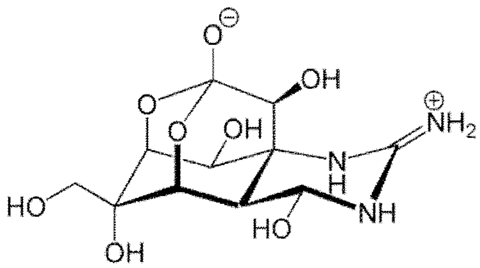

**Formula I:** Chemical structure of Tetrodotoxin ($C_{11}H_{17}N_3O_8$)

#### a. Sources of Tetrodotoxin

Tetrodotoxin has been isolated from animals, such as the blue-ringed octopuses, and is produced by bacteria. The most common bacteria associated with TTX production are *Vibrio* Sp. bacteria, with *Vibrio alginolyticus* being the most common species. Pufferfish, chaetognaths, and nemerteans have been shown to contain *Vibrio alginolyticus* and TTX, however the link between these facts and production of TTX in animals has not been firmly established, and there remains much debate in the literature as to whether the bacteria are truly the source of TTX in animals. Although tetrodotoxin is perhaps the most widely known site 1 toxin, it is expensive for clinical use since it must come from the puffer fish; when the endo-symbiotic bacteria that makes TTX is grown *ex vivo,* its production of TTX diminishes. Tetrodotoxins can be obtained from the ovaries and eggs of several species of puffer fish and certain species of California newts. Numerous schemes for the total chemical synthesis of Tetrodotoxin has also been reported, including by Diels-Alder Reactions or Syntheses of TTX from Carbohydrates and Congeners (Ohyabu, et al., J Am Chem Soc. Jul 23;125(29): pp. 8798-805 (2003)); Nishikawa, et al., Angew. Chem. Int. Ed., 43, 4782. DOI: 10.1002/anie.200460293 (2004); reviewed in Chau and Ciufolini, Mar Drugs, 9(10): 2046-2074 (2011)). Synthesis features rapid construction of the cyclohexene by Diels-Alder cycloaddition using an enantiomerically-pure dienopile, the early introduction of the aminated quaternary center, and the use of that center to direct the relative configuration of further functionalization around the ring.

**ii. Phycotoxins**

Phycotoxins act as a specific blocker of the voltage-dependent sodium channels present in excitable cells (Kao, C.Y., Pharm. Rev., 18: 997-1049 (1966)). Due to the inhibition of sodium channels, the transmission of a nervous impulse is blocked and the release of neurotransmitters is prevented at the level of the neuromotor junction, which prevents muscular contraction. Due to these physiological effects, these compounds are potentially useful in pharmacology when used as muscular activity inhibitors in pathologies associated with muscular hyperactivity, such as muscular spasms and focal dystonias, when applied locally in injectable form. Additionally, since a blockage of the nervous impulse at the transmission level is generated when these compounds are applied as a local infiltration, they are not only able to block the efferent neurotransmission pathways, but also block afferent pathways and cause an inhibition of the sensory pathways and generate an anesthetic effect when these compounds are locally injected. This is a surprising effect, since both effects are simultaneous, as described in U.S. Patent No. 4,001,413.

The chemical structure of these phycotoxins has a general structure of Formula II:

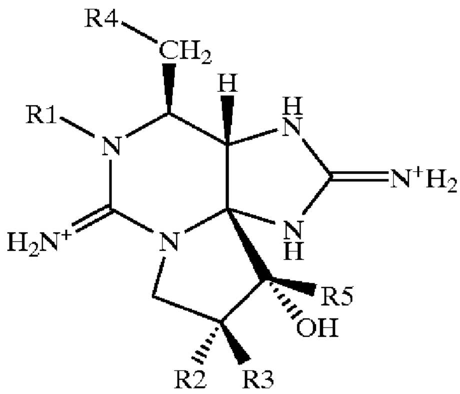

**Formula II:** General chemical structure of the phycotoxins

The particular chemical structure of the structure is defined by the substituents R1 to R5 according to the Table 1.1.

**Table 1.1:** Chemical Structures of Phycotoxins relative to the Formula I

| Compound | R1 | R2 | R3 | R4 | R5 |
|---|---|---|---|---|---|
| Saxitoxin | H | H | H | $COONH_2$ | OH |
| Neosaxitoxin | OH | H | H | $COONH_2$ | OH |
| Gonyaulatoxin 1 | OH | H | $OSO^{-3}$ | $COONH_2$ | OH |
| Gonyaulatoxin 2 | H | H | $OSO^{-3}$ | $COONH_2$ | OH |
| Gonyaulatoxin 3 | OH | $OSO^{-3}$ | H | $COONH_2$ | OH |
| Gonyaulatoxin 4 | H | $OSO^{-3}$ | H | $COONH_2$ | OH |
| Gonyaulatoxin 5 | H | H | H | $COONHSO^{-3}$ | OH |

**a. Saxitoxin**

Saxitoxin (STX) was first extracted from the Alaska butterclam, *Saxidomus gigantcus,* where it is present in algae of the genus Gonyaulax. The reported chemical formula is C10 H15 N7 O3.2HCl. It is freely soluble in water and methanol and it is believed the toxin has a perhydropurine nucleus in which are incorporated two guanidinium moieties. STX is responsible for paralytic shellfish poisoning. It is reported to be one of the most toxic non-protein compounds known, with a toxicity of 8 μg/Kg in mice (approximately 0.2-1.0 mg would prove fatal to humans), and is therefore widely considered too toxic to be used alone as a local anesthetic.

**b. Neosaxitoxin and Decarbamoyl Saxitoxin**

Neosaxitoxin and decarbamoyl saxitoxin are potentially more potent and may have advantages over saxitoxin in formulation.

Neosaxitoxin ("NeoSTX", or "Neo") is under clinical development as a prolonged duration local anesthetic (Rodriguez-Navarro, et al., Anesthesiology, 2007;106:339-45; Rodriguez-Navarro, et al., Neurotox. Res., 2009;16:408-15; Rodriguez-Navarro, et al., Reg. Anesth. Pain Med., 2011;36:103-9). A Phase 1 study of subcutaneous infiltration in human volunteers showed that NeoSTX caused effective cutaneous hypoesthesia (Rodriguez-Navarro, et al., Anesthesiology, 2007; 106:339-45) and a second Phase 1 study showed that combination with bupivacaine resulted in more prolonged analgesia compared to NeoSTX or bupivacaine alone (Rodriguez-Navarro, et al., Neurotox. Res., 2009; 16:408-15).

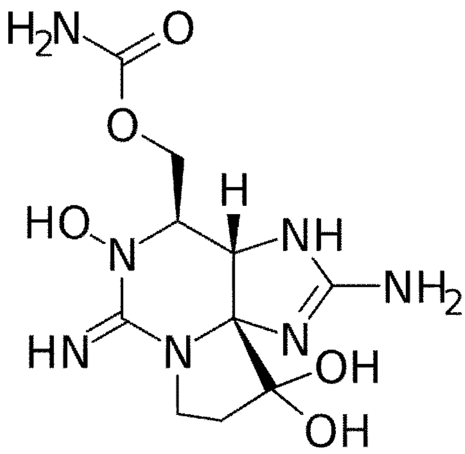

**Formula III:** Neosaxitoxin (Neo)

**(i). Sources of Phycotoxins**

Saxitoxin (STX) and its derivatives can be produced in bioreactors from algae. The phycotoxins neosaxitoxin, saxitoxin and gonyaulatoxins are active compounds produced by harmful algae blooms of the genera *Alexandrium* sp., *Piridinium* sp., and *Gimnodinium* sp. (Lagos, N. Biol. Res., 31: 375-386 (1998)). In the last 15 years, it has been demonstrated that these phycotoxins can also be produced by fresh water cyanobacteria such as photosynthetic blue-green algae, besides being produced by marine dinoflagellates.

Only four genera of cyanobacteria able to produce paralyzing phycotoxins have been identified, and each produces a different mixture of phycotoxins both in amounts and in types of phycotoxins produced, *i.e.* they produce different profiles of paralyzing phycotoxins (Lagos, et al., TOXICON, 37: 1359 - 1373 (1999); Pereira, et al., TOXICON, 38: 1689 - 1702 (2000)).

STX can also be produced by chemical synthesis according to at least three distinct methods (Kishi, et al., J. Am. Chem. Soc., 98, 2818 (1977); Jacobi, et al., J. Am. Chem. Soc., 106 (19), pp 5594-5598 (1984); Fleming, et al., J. Am. Chem. Soc., 3926 (2006)).

Two saxitoxin derivatives, neosaxitoxin (NeoSTX) and decarbamoyl saxitoxin, have advantages in terms of the production process and potency. A study examined rat sciatic nerve blockade with several members of the saxitoxin series, including NeoSTX (Kohane, et al., Reg. Anesth. Pain Med., 25:52-9 (2000)). Saxitoxin and these two derivatives all give markedly synergistic block and prolonged block (1-2 days in rat sciatic nerve *in vivo*) when combined with bupivacaine or epinephrine.

**B. Polymers**

Conjugates for the controlled release of anesthetics *in vivo* include one or more polymers to which the anesthetic is covalently conjugated, for example, by an ester linkage. The polymer to which the anesthetic is bound may also be referred to as the "backbone" polymer.

The covalent conjugate of an anesthetic and a polymer requires a "backbone" formed from a suitable polymer onto which the anesthetic can be bound. The polymer backbone comprises a di-carboxylic acid/Triol (TD) polymer. Representative schemes for the production and conjugation of such polymers are provided in Figures 1A and 1B,

respectively.

In some embodiments, the conjugates include a polyester polymer. In some embodiments, the conjugates include blends of more than one polyester polymer. A preferred polyester polymer is poly(glycerol sebacate) (PGS).

In some embodiments, PGS is blended with one or more additional polyester polymers to alter one or more of the material properties of the conjugate. Suitable polyester polymers for including within the conjugates include bioresorbable synthetic polyesters, such as poly(lactide-co-caprolactone), poly(caprolactone) (PCL), polydioxanone (PDO), poly(ortho) esters, poly(glycolic acid) (PGA), polymers of lactic acid and glycolic acid, poly(lactic acid) (PLA), polybutyrate), poly(lactide-co-glycolide), poly(hydroxybutyrate), and copolmyers, blends, and chemical derivatives thereof (substitutions, additions of chemical groups, for example, alkyl, alkylene, hydroxylations, oxidations, and other modifications routinely made by those skilled in the art). Any natural or synthetic polymer having -COOH or -OH groups can be used to conjugate anesthetic agents. Therefore, natural and synthetic polymers such as hyaluronic acid, tannic acid, poly-acrylic acid and other compounds including a carboxyl terminal or side chain moiety can be included in anesthetic conjugates. Generally, polymers included in the covalent anesthetic conjugates are biodegradable and biocompatible. However, in some embodiments, one or more non-biodegradable polymers are included covalent anesthetic conjugates. When covalent anesthetic conjugates include blends of one or more biodegradable and one or more non-biodegradable polymers, the proportion of the biodegradable polymer to the non-biodegradable polymer can be varied according to the needs of the recipient, for example, to tune the amount and/or rate of delivery of the anesthetic agent(s).

**1. Poly(glycerol sebacate) (PGS)**

In some embodiments, other bioresorbable polyesters are combined with, or substituted for, poly(glycerol sebacate) (PGS) for use in covalent anesthetic-polymer conjugates. Conjugates of anesthetics covalently bound to polymers for controlled release of anesthetics *in vivo* can include poly(glycerol sebacate) (PGS). PGS is a biodegradable polyester polymer used in a variety of biomedical applications. PGS is typically prepared by polycondensation of glycerol and sebacic acid. PGS is biocompatible and biodegradable, and the mechanical properties and degradation kinetics of PGS can be tailored by controlling factors such as curing time, curing temperature, concentration of reactants, and the degree of acrylation in acrylated PGS (Rai, et al., Progress in Polymer Science V. 37, (8), pp. 1051-1078 (2012)).

Glycerol is the basic building block for lipids, while sebacic acid is the natural metabolic intermediate in w-oxidation of medium- to long-chain fatty acids. In addition, glycerol and copolymers containing sebacic acid have been approved for their medical applications by the Food and Drug Administration (FDA). PGS has been proposed for tissue engineering applications due to its desirable mechanical properties, biocompatibility and controlled degradation (Jia, Y. et al. Polymer Chemistry 7, 2553-2564, doi:10.1039/c5py01993a (2016); Rai, et al. Progress in Polymer Science 37, 1051-1078 (2012); Wang, et al. Journal of Biomedical Materials Research Part A 66A, 192-197, (2003); Loh, et al. Journal of Materials Chemistry B 3, 7641-7652 (2015); Wang, et al. Nat Biotech 20, 602-606 (2002)). PGS terminates with hydroxyl groups and carboxyl groups, and can be readily covalently conjugated to peptides, proteins and drugs. TTX, with a molecular structure rich in hydroxyl groups, can be covalently conjugated onto PGS via ester bonds, and released *via* the hydrolysis of these bonds, as depicted in Figure 1. In addition, the surface erodible nature of PGS and PGS-based copolymers, which induces a long-term, consistent release of TTX following a near zero-order profile, makes them unique and preferable to other polyesters for controlled drug delivery applications (Wang, et al. Journal of Biomedical Materials Research Part A 66A, 192-197 (2003)). The incorporation of PEG allows for tuning of the hydrophilicity of the resulting conjugates, and therefore their rate of TTX release. These characteristics confer on this conjugate the potential for prolonged duration local anesthesia with minimal systemic and local toxicity.

**2. Polymers for Tunable Hydrophilicity**

Conjugates of anesthetics covalently bound to poly(glycerol sebacate) (PGS) optionally include one or more additional hydrophilic polymers. The hydrophilic polymers extend the PGS backbone and vary the hydrophilicity of the conjugate. Increasing the relative amount of hydrophilic polymer to the PGS increases the hydrophilicity of the conjugate. The hydrophilicity of the conjugate is related to the rate of hydrolysis of ester bonds *in vivo*. The rate of hydrolysis of ester bonds in the PGS-anesthetic conjugates is related to the release of the anesthetic agent from the conjugate *in vivo.* Therefore, the rate of release of the covalently-bound anesthetic from the polyester conjugate *in vivo* is controlled by the amount and size of the hydrophilic polymer attached to or associated with the PGS-anesthetic conjugate.

In preferred embodiments, the one or more hydrophilic polymer component contains a poly(alkylene glycol) chain. The poly(alkylene glycol) chains may contain between 1 and 500 repeat units, more preferably between 40 and 500 repeat units. Suitable poly(alkylene glycols) include polyethylene glycol, polypropylene 1,2-glycol, poly(propylene oxide), polypropylene 1,3-glycol, and copolymers thereof.

A wide variety of hydrophilic polymers can be included, including poly β-amino esters and 1, 2-amino alcohol lipids. In some embodiments, the polymers are alkyl-modified polymers, such as alkyl modified poly(ethylene glycol).

Other exemplary polymers include poly(alkylene glycol), polysaccharides, poly(vinyl alcohol)s, polypyrrolidones, polyoxyethylene block copolymers (*e.g.*, PLURONIC®), polyethylene glycol (PEG) and copolymers thereof. Preferred hydrophilic polymers are biocompatible (i.e., do not induce a significant inflammatory or immune response) and non-toxic. Examples of suitable hydrophilic polymers include, but are not limited to, poly(alkylene glycols) such as polyethylene glycol (PEG), poly(propylene glycol) (PPG), and copolymers of ethylene glycol and propylene glycol, poly(oxyethylated polyol), poly(olefinic alcohol), polyvinylpyrrolidone), poly(hydroxyalkylmethacrylate), poly(hydroxyalkylmethacrylamide), poly(saccharides), poly(amino acids), poly(vinyl alcohol), and copolymers, terpolymers, and mixtures thereof.

In some embodiments, the one or more hydrophilic polymer components are copolymers containing one or more blocks of polyethylene oxide (PEO) along with one or more blocks composed of other biocompatible polymers (for example, poly(lactide), poly(glycolide), poly(lactide-co-glycolide), or polycaprolactone). The one or more hydrophilic polymer segments can be copolymers containing one or more blocks of PEO along with one or more blocks containing polypropylene oxide (PPO). Specific examples include triblock copolymers of PEO-PPO-PEO, such as POLOXAMERS™ and PLURONICS™.

**a. Poly(ethylene glycol) (PEG)**

Conjugates of anesthetics covalently bound to (PGS) optionally including one or more PEGs are described.

PEG is a commonly used hydrophilic polymer. The size, relative quantity and distribution of the amphiphilic PEG included in the MDNPs can influence the biophysical characteristics of the resulting modified dendrimer-based nanoparticle (MDNPs), such as structural features and charge density.

The physical properties of the MDNPs is directly associated with the size, relative quantity and distribution of the amphiphilic PEG (*i.e.,* the extent of pegylation). Exemplary properties that can be modified include the efficacy of uptake of the MDNPs by one or more types of eukaryotic cells, the speed and efficacy of the intracellular delivery of therapeutic, prophylactic and diagnostic agents, and the immunogenicity and cytotoxicity of the MDNP. In certain embodiments, pegylation results in charge neutralization of the MDNP.

Typically, the amphiphilic PEG includes a short-chain oligo-ethylene glycol. Exemplary oligoi-ethylene glycols include di-ethylene glycol, tri-ethylene glycol, tetraethylene glycol, penta-ethylene glycol, and hexa-ethylene glycol.

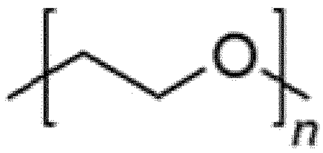

**Formula VIII:** Repeating unit of a short chain oligo-ethylene glycol (n=1-6) PEG monomers.

In some embodiments, the amphiphilic polymer is a phospholipid conjugated to monomethoxy polyethyleneglycol (mPEG). In certain embodiments, the lipid-associated PEG or mPEG is a branched or "multi-arm" PEG. MDNPs can include multiarm polyethylene glycol having at least two branches bearing sulfhydryl or thiopyridine terminal groups; however, PEG polymers bearing other terminal groups such as succinimidyl or maleimide terminations can be used.

The covalent anesthetic-polymer conjugates can include polyethylene glycol polymers having different molecular weights. For example, the PEGs can have molecular weights between approximately 100 Da (*i.e.,* PEG 100 Da) and approximately 12,000 kDa (*i.e.,* PEG 12 KDa), inclusive. Covalent anesthetic-polymer conjugates can be formed using a single species of amphiphilic PEG, or from two or more different species of amphiphilic PEGs. For example, covalent anesthetic-polymer conjugates can be formed with multiple different species of PEGs having different molecular weights.

Covalent anesthetic-polymer conjugates can be formed using a single amphiphilic polymer species, or a mixture of multiple different amphiphilic polymer species. The amphiphilic polymers can be modified with adducts. For example, amphiphilic polymers can be modified with the same or different the one or more different adducts. Therefore, covalent anesthetic-polymer conjugates can be formed using PGS and one or more PEGS and other polymers, optionally including mixtures of the same or different adducts.

Exemplary PEGs are those having a molecular weight such as PEG(100); PEG(200); PEG(300); PEG(400); PEG(500); PEG(600); PEG(750); PEG(800); PEG(900); PEG(1,000); PEG(2,000); PEG(3,000); PEG(5,000); PEG(6,000); PEG(7,000); PEG(8,000); PEG(9,000); PEG(10,000); PEG(12,000); and PEGs having a molecular weight greater than 12,000, such as PEG(20,000). The lipidic component can include saturated or non-saturated fatty acidic moieties.

In some embodiments, covalent anesthetic-polymer conjugates include one or more species of PEG or

[methoxy(polyethylene glycol)] mPEG) molecules. When more than a single species of PEG and/or mPEG are employed, they may be present in the same or different molar ratios. Exemplary PEG or mPEGs that can be incorporated within covalent anesthetic-polymer conjugates include a PEG or mPEG component with a molecular weight of between 100 Da and 80,000 Da, for example, between 100 Da and 10,000 Da, more preferably between 200 Da and 5,000 Da, most preferably between 200 and 2,000 Da.

PEGs and/or mPEGs can be included at a molar ratio of polyester to PEG of between 1:01 and 1:1,000. For example, in some embodiments, the PEG is present in a PGS-PEG-TTX conjugate at a molar ratio of 1:1 PGS:PEG, or greater than 1:1, such as 1:2, 1:3, 1:4; 1:5, 1:6, 1:7, 1:8, 1:9 or 1:10, or greater than 1:10. In some embodiments, the PEG is present in a PGS-PEG-TTX conjugate at a molar ratio of 1:1 PEG:PGS, or greater than 1:1, such as 1:2, 1:3, 1:4; 1:5, 1:6, 1:7, 1:8, 1:9 or 1:10, or greater than 1:10.

The amount and size of PEG (or other hydrophilic polymer) included within the conjugates can be varied according to the desired physiological properties of the conjugate *in vivo.*

### C. Covalent Linker Moieties

Conjugates of anesthetics covalently bound to PGS, or PGS-PEG, or other polymer conjugates bind the one or more anesthetic agents through one or more types of covalent linkages. In preferred embodiments, the covalent linkages are biodegradable linkages, such that the linkage is degraded or otherwise broken *in vivo,* releasing the anesthetic agent(s) at or near the site of degradation. An exemplary biodegradable covalent linkage is a hydrolysable bond, such as an ester bond.

In some embodiments, covalent anesthetic-polymer conjugates include anesthetic agents and/or additional active agents conjugated or attached directly to PGS, or PGS-PEG, or other polymer backbones, for example, *via* ester-linkages. Optionally, the anesthetic agents or compounds are conjugated to the PGS, or PGS-PEG or other polymer backbone *via* one or more spacers/linkers via different linkages such as disulfide, carbonate, carbamate, thioester, hydrazine, hydrazides, and amide linkages. In some embodiments, the attachment occurs *via* an appropriate spacer that provides a disulfide bridge between the agent and the PGS, or PGS-PEG or other polymer backbone. In this case, the covalent anesthetic-polymer conjugates are capable of rapid release of the agent *in vivo* by thiol exchange reactions, under the reduced conditions found in body.

#### 1. Spacers

In some embodiments, the anesthetic agents are coupled to the polymer backbone via one or more intermediate "spacer" or "linker" molecules. The spacers or linkers can enhance the hydrophilicity of the molecule, and thereby enhance the rate of hydrolysis or degradation of the anesthetic/polymer linkage, resulting in enhanced release of the anesthetic agent *in vivo.* Therefore, spacers can be included to modify the rate of release of the anesthetic and/or additional active agent from the polymer *in vivo.*

The term "spacers" includes compounds used for linking an active agent to the PGS, or PGS-PEG or other polymer backbone. The spacer can be either a single chemical entity or two or more chemical entities linked together to bridge the polymer and the anesthetic agent. The spacers can include any small chemical entity, peptide or polymers having sulfhydryl, thiopyridine, succinimidyl, maleimide, vinylsulfone, and carbonate terminations.

The spacer can be chosen from among compounds terminating in sulfhydryl, thiopyridine, succinimidyl, maleimide, vinylsulfone and carbonate group. The spacer can include thiopyridine terminated compounds such as dithiodipyridine, N-Succinimidyl 3-(2-pyridyldithio)-propionate (SPDP), succinimidyl 6-(3-[2-pyridyldithio]-propionamido) hexanoate (LC-SPDP) or sulfosuccinimidyl 6-(3-[2-pyridyldithio]-propionamido)hexanoate (Sulfo-LC-SPDP). The spacer can also include peptides wherein the peptides are linear or cyclic essentially having sulfhydryl groups such as glutathione, homocysteine, cysteine and its derivatives, arg-gly-asp-cys (RGDC), cyclo(Arg-Gly-Asp-d-Phe-Cys) (c(RGDfC)), cyclo(Arg-Gly-Asp-D-Tyr-Cys), cyclo(Arg-Ala-Asp-d-Tyr-Cys). The spacer can be a mercapto acid derivative such as 3 mercapto propionic acid, mercapto acetic acid, 4 mercapto butyric acid, thiolan-2-one, 6 mercaptohexanoic acid, 5 mercapto valeric acid and other mercapto derivatives such as 2 mercaptoethanol and 2 mercaptoethylamine.

In some embodiments, the spacer is thiosalicylic acid and/or its derivatives, (4-succinimidyloxycarbonyl-methyl-alpha-2-pyridylthio)toluene, (3-[2-pyridithio]propionyl hydrazide. In some embodiments, the spacer has maleimide terminations wherein the spacer includes polymer or small chemical entities such as bis-maleimido diethylene glycol and bis-maleimido triethylene glycol, Bis-Maleimidoethane, bismaleimidohexane. In some embodiments, the spacer includes vinylsulfone such as 1,6-Hexane-bis-vinylsulfone. In some embodiments, the spacer includes thioglycosides such as thioglucose. In some embodiments, the spacer is formed from one or more reduced proteins, such as bovine serum albumin and human serum albumin, any thiol terminated compound capable of forming disulfide bonds. The spacer can also include polyethylene glycol having maleimide, succinimidyl and thiol terminations.

In some embodiments, the spacer/linker is Gamma-aminobutyric acid (GABA) linker, allyl linker, propargyl linker,

ethane thiol linker, pyridine disulfide linker. In some embodiments, the spacer/linker is conjugated to the PGS, or PGS-PEG or other polymer backbone *via* one or more of ether, thioester, carbamate, carbonate, hydrazine, or amide bonds for improved stability under physiological conditions, for example, compared to ester linkages.

In other embodiments, the ligation of different linkers *e.g.,* allyl, propargyl, *etc.* on PGS, or PGS-PEG or other polymer backbone surface through different linkages *e.g.,* ether, ester, carbamate, carbonate *etc.,* which can participate in click chemistry for the conjugation of the active agent such as NAC.

In further embodiments, the PGS, or PGS-PEG or other polymer backbone is conjugated to a first anesthetic or active agent *via* one linker, whilst a second anesthetic or active agent *via* a different linker.

**D. Penetration Enhancing Excipients**

It has been established that polyethylene oxide functions as a chemical penetration enhancer (CPE) for the anesthetic-polymer conjugates.

The data in the Examples demonstrate that PEG200 functions as a CPE when anesthetic-polymer conjugates are dispersed into a solution of the PEG200. Solutions of anesthetic-polymer conjugates dispersed in PEG can enhance drug flux into the nerve, which could attributed to the amphipathic nature of PEG200. It may be that the CPE enables or facilitates the ability of the anesthetic to cross the perineurial barrier to enhance drug flux into nerves.

As described in the Examples, below, non-conjugated anesthetic (e.g., TTX), at doses from 1 to 3 $\mu$g, in 0.5 mL of PEG200 resulted in complete nerve blockade in 100% of test animals, with a block duration up to 5.3$\pm$0.3 hours; this is three times longer than the effect of 0.5% bupivacaine - an anesthetic commonly used in the clinic. Being low toxicity and already widely used in a variety of pharmaceutical formulations, PEG200 is a suitable delivery medium for local anesthesia in the clinic.

Therefore, in some embodiments, formulations of anesthetic-polymer conjugates include an excipient that contains PEG. A preferred excipient is PEG200. The excipient enhances the efficacy of the anesthetic-polymer conjugates.

With a good miscibility, TDP-TTX conjugates were readily dispersed in PEG200, forming a homogeneous, syringe injectable TDP-TTX/PEG200 formulation. All TDP-TTX/PEG200 formulations prepared behaved as a liquid with a viscosity less than 10 Pa.s, enabling administration into patients through syringe-injection.

In some embodiments, the anesthetic-polymer conjugates and CPE are integrated in one system. In an exemplary embodiment the inclusion of PEG200 within a formulation of PGS-PEG-TTX has a broader therapeutic index than an equal amount of PGS-PEG-TTX in the absence of the PEG200 excipient.

Therefore, in some embodiments, conjugates of anesthetics covalently bound to PGS, or PGS-PEG are dispersed in PEG, such as PEG200, to enhance the efficacy of the injectable formulation.

In preferred embodiments, covalent anesthetic conjugates are formulated as a liquid for administration directly onto or adjacent to a painful site *via* injection or infiltration. Preferred excipients include polyethylene glycol (PEG) having a molecular weight of between 1Da and 1,000 Da, such as PEG 200.

**1. Syringe-Injectable Formulations**

The choice of solvent for syringe-injectable formulations of PGS-TTX and PGS-PEG-TTX must consider the desired specific flow properties needed for proper administrated to the affected area by patients (Mastropietro, et al. J Dev Drugs 2, 108 (2013)). For example, PGS is a thermoset polymer, and the elastic modulus of PGS can be easily tuned by controlling various parameters such as reaction time, reaction temperature and time of curing.

In some embodiments, the covalent anesthetic-polymer conjugates include a sufficient quantity of hydrophilic polymer to enable the conjugate to be dispersed in an aqueous medium, such as phosphate buffered saline (PBS). For example, PGS-PEG2000-TTX and PGS-PEG1000-TTX conjugates with a higher percentage of PEG in the backbone can be homogeneously suspended in PBS. However, some PGS-PEG-TTX and PGS-TTX conjugates cannot readily be homogeneously suspended in PBS. Therefore, in some embodiments, a solubilizing agent is added to increase the hydrophilicity of the conjugates, amenable to an injectable formulation. Exemplary solubilizing agents include PEG. A preferred PEG is PEG200. It has been established that PEG200 not only functions as a solubilizing medium for the conjugates, but also functions as a penetration enhancing agent to enhance the effectiveness of the conjugates as delivery vehicles for anesthetic agents.

**i. PEG**

In some embodiments covalent anesthetic conjugates are formulated as a syringe-injectable form by homogeneous suspension in PEG.

The term "injectate" refers to a material that is to be injected. In some embodiments the injectate includes one or

more excipient material that enable or enhance the dispersion of the covalent anesthetic conjugates as fluid medium, amenable to administration via injection or infiltration. Therefore, in some embodiments, covalent anesthetic conjugates are formulated as an injectable solution by dispersion in an aqueous solution of PEG.

Typically, PEG is added to the formulation, to increases hydrophilicity of PGS-PEG-TTX conjugates. The increase in PEG concentration renders dissolution of copolymer in PBS. For example, in some embodiments, PGS-TTX conjugates are homogeneously suspended in PEG100, PEG-monomethyl-ether (mPEG), PEG200, PEG250, PEG300, PEG400 or PEG500.

In a preferred embodiment, covalent anesthetic conjugates are formulated as a syringe-injectable form by homogeneous suspension in PEG200. PEG 200 is a clear, colorless, viscous liquid. Due in part to its low toxicity, PEG 200 is widely used in a variety of pharmaceutical formulations. To make a high-quality and syringe-injectable formulation, PGS-PEG-TTX and PGS-TTX conjugates are formulated into PEG 200 *via* a solvent evaporation process, as depicted by the diagram in Figure 4.

The viscoelastic properties of the synthesized formulations were investigated via rheology. For the formulation of PGS-PEG200-TTX/PEG200 (50 mg/ml), the loss modulus (G") was higher than the storage modulus (G'), indicating the viscous component of the complex modulus dominates the material behavior (Figure 5A). In addition, although the viscosity increased as the PEG concentration in the conjugate decreased, all PGS-PEG-TTX/PEG200 formulations (50 mg/ml) had a viscosity less than 10 Pa.s under the angular frequency tested (Figure 5B). With viscous behavior and low viscosity, PGS-PEG-TTX/PEG200 formulations were all syringe-injectable.

### E. Covalent Anesthetic-Polymer Conjugates and Dosage Units

Conjugates of anesthetics covalently bound to hydrophilic polymers such as PGS, or PGS-PEG, include (i) an effective amount of local anesthetic to provide nerve blockade; (ii) a hydrophilic polymer backbone (preferably PGS); and (iii) optionally one or more PEGs bound to the polymer backbone in the relative ratios required to provide the desired functional elements of (a) total amount of anesthetic loaded per unit dose of the formulation; and (b) rate of release of the anesthetic from the polymer backbone *in vivo.*

The rate of release and the dose per unit time period of the anesthetic (*i.e.,* dose/hour, dose/day, *etc.*) can be varied as a function of the total amount administered, as well as the ratio of the hydrophilic polymers to one another (*e.g.,* the ratio of the amount of PGS to the amount of PEG). For example, in some embodiments, the addition of PEG into a polymer-anesthetic conjugate determines the overall hydrophilicity of the conjugate. The overall hydrophilicity of the conjugate determines the hydrolysis rate of ester bonds *in vivo.* The hydrolysis rate of ester bonds *in vivo* determines the release rate of the anesthetic *in vivo.* Therefore, the overall hydrophilicity of the conjugates can be tuned by addition of more or less PEG to reduce or prolong the period of time over which the hydrolysis of the ester-bonds within the conjugate occurs *in vivo.*

In some embodiments, the anesthetic release rate *in vivo* is adjusted by tuning the percentage of PEG segment in the polymer-anesthetic conjugates to provide release of the active agent for a period of up to one month following exposure to physiological conditions *in vivo.* For example, the rate of release of TTX *in vivo* can be adjusted by tuning the percentage of PEG segment in the PGS-TTX conjugates. In some embodiments the rate of release of TTX is increased in proportion to the molecular weight of the PEG incorporated in the PGS-PEG backbone. Therefore, TTX-PGS-PEG1000 will release TTX *in vivo* at a much higher rate than an equal molar amount of TTX-PGS-PEG100.

Conjugates can be formulated such that the rate of release *in vivo* is on the order of 50%, 60%, 70%, 80%, 90% or more than 90% of the total anesthetic is released in a period of 1-30 days. In an exemplary embodiment, after 28 days, 99.7% of the TTX is released from PGS-PEG2000-TTX; whereas PGS-PEG1000-TTX, PGS-PEG200-TTX, and PGS-TTX indicated 89.5, 62.5, and 43.7% TTX release, over the same time period, respectively. In the case of the PGS-PEG200-TTX and PGS-TTX conjugates, TTX is released following a zero-order release profile, which is attributed to the degradation of PGS-based copolymers under a surface erosion mechanism. The increase in TTX release with an increase in PEG concentration is attributed to an increase in the hydrophilicity of the copolymer backbone. Such increase in hydrophilicity results in greater water uptake, which accelerates the hydrolysis of ester bonds. The results presented in the Examples confirm that drug release occurs due to the hydrolysis of ester bonds. The tunable sustained release of the compounds of interest for several weeks supports the conjugates' ability to provide prolonged duration local anesthesia.

In an exemplary embodiment, conjugates of PEG-PGS include an excess molar ratio of PGS over PEG. For example, the molar ratio between the -COOH group and - OH for PGS-PEG copolymer synthesis is typically 8:7. The remaining -COOH groups are conjugated to anesthetics and optionally additional active agents (see Tables 1, and 2). The ratio of free "-COOH" to free "-OH" groups in the PGS/polymer mixture can be used to calculate the total amount of PEG that is incorporated into the PGS. Typically, the design of conjugates allows for incorporation of at least one anesthetic and optionally an additional active agent.

### F. Additional Agents

Formulations of covalent anesthetic-polymer conjugates can include one or more additional pharmaceutically active agents. Active agents, such as therapeutic, prophylactic and/or diagnostic agents can be associated with covalent anesthetic-polymer conjugates. In some embodiments, the active agents are covalently or non-covalently associated with the covalent anesthetic-polymer conjugates. For example, additional active agents can be covalently bound to the polymer backbone *via* ester-bonds. In other embodiments, additional active agents are bound *via* non-ester covalent linkers to the polymer backbone, for example, *via* one or more spacers. When active agents are covalently associated with the polymer backbone, the agents can be released from the backbone *in vivo* at a time that is consistent with, or different to, the timing of the release of the covalent anesthetic *in vivo.* Therefore, in some embodiments, formulations including covalent anesthetic-polymer conjugates deliver both the anesthetic and one or more additional active agents at the same or different rates throughout the same or different time *in vivo.* In some embodiments, additional active agents are non-covalently associated with the anesthetic-polymer conjugates. For example, formulations of covalent anesthetic-polymer conjugates can include on or more additional active agents by admixture. Exemplary additional active agents include additional local anesthetic agents, vasoconstrictors, hormones, anti-inflammatories and anti-infective agents.

#### 1. Local Anesthetic Agents

Formulations of covalent anesthetic-polymer conjugates can include one or more additional local anesthetic agents. The term "local anesthetic" means a drug which provides local numbness or pain relief. Classes of local anesthetics which can be utilized include the aminoacylanilide compounds such as lidocaine, prilocaine, bupivacaine, mepivacaine and related local anesthetic compounds having various substituents on the ring system or amine nitrogen; the aminoalkyl benzoate compounds, such as procaine, chloroprocaine, propoxycaine, hexylcaine, tetracaine, cyclomethycaine, benoxinate, butacaine, proparacaine, and related local anesthetic compounds; cocaine and related local anesthetic compounds; amino carbonate compounds such as diperodon and related local anesthetic compounds; N-phenylamidine compounds such as phenacaine and related anesthetic compounds; N-aminoalkyl amid compounds such as dibucaine and related local anesthetic compounds; aminoketone compounds such as falicaine, dyclonine and related local anesthetic compounds; and amino ether compounds such as pramoxine, dimethisoquien, and related local anesthetic compounds. The preferred local anesthetics are amino-amides and amino esters, with the most preferred being bupivacaine, the levoenantiomer of bupivacaine being preferred where vasoconstrictor activity of the local anesthetic is desirable, tetracaine, and ropivacaine, which is slightly more sensory selective.

These drugs average six to ten hours of pain relief when given in different sites and for different types of surgery. For many types of surgery, it would be preferable to have durations of pain relief that last two or three days. The preferred local anesthetics for use in combination with NeoSTX are bupivacaine, ropivacaine, tetracaine and levobupivacaine. Bupivacaine is a particularly long acting and potent local anesthetic. Its other advantages include sufficient sensory anesthesia without only partial motor blockade, and wide availability.

#### 2. Vasoconstrictors

Formulations of covalent anesthetic-polymer conjugates can include one or more vasoconstrictor agents. Vasoconstrictors which are useful are those acting locally to restrict blood flow, and thereby retain the injected drugs in the region in which they are administered. This has the effect of substantially decreasing systemic toxicity.

Preferred vasoconstrictors are those acting on alpha adrenergic receptors, such as epinephrine and phenylepinephrine. Other drugs and dyes vasoconstrict as a side-effect, such as bupivacaine and levobupivacaine.

In some embodiments, conjugates of anesthetics covalently bound to PGS, or PGS-PEG, or other polymer conjugates are formulated with epinephrine. For example, epinephrine may be included in an amount effective to enhance or prolong the nerve blockade resulting from the one or more anesthetic agents within the conjugate.

#### 3. Glucocorticoids

Formulations of covalent anesthetic-polymer conjugates can include one or more glucocorticoids. Exemplary glucocorticoids include dexamethasone, cortisone, hydrocortisone, prednisone, beclomethasone, betamethasone, flunisolide, methyl prednisone, para methasone, prednisolone, triamcinolome, alclometasone, amcinonide, clobetasol, fludrocortisone, diflurosone diacetate, fluocinolone acetonide, fluoromethalone, flurandrenolide, halcinonide, medrysone, and mometasone, and pharmaceutically acceptable salts and mixtures thereof.anti-inflammatory agents. In some embodiments, conjugates of anesthetics covalently bound to PGS, or PGS-PEG, or other polymer conjugates are formulated with dexamethasone. Dexamethasone has a hydroxyl group that allows it to be covalently conjugated onto PGS-PEG *via* the same ester bond used in the conjugation of TTX. As described in the Examples, the drug release profiles

for TTX and dexamethasone were consistent for all PGS-PEG-drug conjugates. Mass loss followed a similar trend to that of drug release for PGS-PEG-TTX conjugates (Figures 3A-3D). After 28 days, PGS-TTX had a 27.3% mass loss, whereas PGS-PEG200-TTX, PGS-PEG1000-TTX and PGS-PEG2000-TTX had a mass loss of 30.4, 70.4 and 94.7%, respectively.

**4. Other Therapeutic Agents**

In some embodiments, conjugates of anesthetics covalently bound to PGS, or PGS-PEG, or other polymer conjugates are formulated with additional active agents, such as anti-inflammatories, anti-infective agents, and anti-proloferative agents. Exemplary anti-inflammatory drugs include nonsteroidal drugs (e.g., indomethacin, aspirin, acetaminophen, diclofenac sodium and ibuprofen); and steroidal anti-inflammatory drug (e.g., dexamethasone)

**G. Other Excipients and Preservatives**

Conjugates of anesthetics covalently bound to PGS, or PGS-PEG, or other polymers can be formulated to include one or more excipients or preservatives agents for administration. Typically, the compositions are formulated to include excipients for administration *via* parenteral routes. Exemplary pharmaceutical compositions of covalent anesthetic conjugates are formulated for administration via intramuscular, intraperitoneal, intravenous (iv), or subcutaneous injection, transdermal administration (either passively or using iontophoresis or electroporation).

Formulations of anesthetics covalently bound to PGS, or PGS-PEG include one or more additional pharmaceutically acceptable excipients. Exemplary additional excipient agents include preservatives, pH adjusting agents, antioxidants, and isotonicity agents. In some embodiments, covalent anesthetic conjugates are formulated in saline, or an acidic buffered solution, optionally containing a preservative, for example, for administration *via* parenteral routes. Pharmaceutical compositions of covalent anesthetic conjugates formulated for administration by parenteral (intramuscular, intraperitoneal, intravenous (iv) or subcutaneous injection), transdermal (either passively or using iontophoresis or electroporation), transmucosal (nasal, vaginal, rectal, or sublingual) routes of administration or using bioerodible inserts and can be formulated in unit dosage forms appropriate for each route of administration.

In some embodiments the covalent anesthetic-polymer conjugates are formulated in an aqueous solution, for administration by parenteral injection. The formulation may also be in the form of a suspension or emulsion. In general, pharmaceutical compositions are provided including effective amounts of covalent anesthetic-polymer conjugates and optionally include pharmaceutically acceptable diluents, preservatives, solubilizers, emulsifiers, adjuvants and/or carriers. Such compositions include diluents such as sterile water, buffered saline of various buffer content (*e.g*., Tris-HCl, acetate, phosphate), pH and ionic strength; and optionally, additives such as detergents and solubilizing agents (*e.g*., TWEEN®20, TWEEN®80, Polysorbate 80), anti-oxidants (*e.g*., ascorbic acid, sodium metabisulfite), and preservatives (*e.g.,* Thimersol, benzyl alcohol) and bulking substances (*e.g.,* lactose, mannitol).

Examples of non-aqueous solvents or vehicles are propylene glycol, polyethylene glycol, vegetable oils, such as olive oil and corn oil, gelatin, and injectable organic esters such as ethyl oleate. The formulations may be lyophilized and redissolved/resuspended immediately before use. The formulation may be sterilized, for example, by filtration through a bacteria retaining filter, by incorporating sterilizing agents into the formulations, by irradiating the formulations, or by heating.

In some embodiments, the covalent anesthetic-polymer conjugates are formulated in a solution with one or more proteins or polypeptides. In an exemplary embodiment, the covalent anesthetic-polymer conjugates are formulated in a solution with the protein Albumin. Albumin proteins, such bovine albumin, can be added to mixtures of the covalent anesthetic-polymer conjugates, for example, to enable or enhance solubilization or dispersion of the conjugates in aqueous solution.

**H. Dosage Forms**

In preferred embodiments, the covalent anesthetic-polymer conjugates are provided in vials in an aqueous solution. Depending on the type of formulation, as outlined previously and below, the vial sizes may range from 10 μl to 200 ml, preferably from about 100 μl to about 10 ml, and 1-5 vials may be used for a single patient in different situations. In another embodiments, covalent anesthetic-polymer conjugates are provided in one or more vials, optionally lyophilized, then rehydrated and combined prior to use.

Preferably, the dosage forms include an amount of anesthetic effective to reduce or prevent pain in a subject in need thereof in the absence of toxicity. In animal studies, 350-400g rats were administered covalent anesthetic-polymer conjugates including approximately 80 μg of conjugated TTX without any systemic toxicity. Dosage forms for administration to humans including covalent anesthetic-polymer conjugates having up to and including 1.5 mg of conjugated TTX are contemplated.

### III. Methods of Use

Methods of using covalent anesthetic-polymer conjugates to deliver anesthetic agents are provided. Methods of providing nerve blockade in a subject in need thereof by administering covalent anesthetic-polymer conjugates to the subject are provided.

The methods can include contacting the tissue surrounding one or more nerves with an effective amount of a covalent anesthetic-polymer conjugates to decrease or inhibit sensory activity in the nerves compared to a control. The methods can prolong the blockade of Site One Sodium Channels (S1SCs) with minimal or reduced toxicity.

In some embodiments the active agents bind to or otherwise inhibit the activity of cell-surface receptors associated with the physiological processes of sensory nerve activity. For example, the anesthetic agents can be effective to block ion channels, such as Site One Sodium Channels (S1SCs), or such as the TRPV1 receptor. In a preferred embodiment the anesthetic agent covalently coupled to the one or more polymers is the S1SCB Tetrodotoxin (TTX). Methods including contacting one or more nerves with an effective amount of TTX to decrease or inhibit sensory activity in the nerves compared to a control are provided. The anesthetic agent is administered as a covalently-bound polymer conjugate, and is subsequently released *in vivo* at or near the site of administration by degradation of the covalent linkage to the polymer backbone.

The methods can consistently release an amount of anesthetic agent in a physiologically active state to achieve effective nerve blockade at the site of administration over an extended period of time. Methods of administering covalent anesthetic-polymer conjugates can provide effective nerve blockade for significantly prolonged periods of time relative to that achieved by administering equivalent amounts of the anesthetic to the same site in the absence of the polymer conjugate. Therefore, methods of administering site one sodium channel blockers as covalent-polymer conjugates *in vivo* can effectively block site 1 sodium channels for a prolonged period of time relative to the equivalent amount of site 1 sodium channel blockers administered to the same site alone. Typically, the methods do not give rise to vasoconstriction.

The covalent anesthetic-polymer conjugates for use in the methods can be formulated such that the anesthetic is released over a period of time from several minutes to several hours, days, weeks, or months. In some embodiments, the methods enable the safe administration of an amount of anesthetic agent that would be toxic to the recipient if administered in the absence of a covalent-polymer conjugate. For example, when the covalent-anesthetic-polymer conjugates are formulated for delivery of the anesthetic at the site of administration over a prolonged period of time, serum concentration of free anesthetic at any given time will be below that when the anesthetic is administered alone as a bolus.

Therefore, in some embodiments, the methods provide prolonged nerve blockade from TTX with reduced toxicity. Typically, the anesthetic agents are released from the polymer backbone *in vivo* in a native form (*i.e.,* in a physiologically active state). In some embodiments, the anesthetic agents are physiologically inactive when covalently conjugated to the polymer backbone.

#### A. Methods for treating or preventing pain

Methods of providing sensory and/or motor nerve blockade in a subject including administering to a subject to or near to a nerve an effective amount of one or more anesthetic agents covalently conjugated to a polymer backbone to reduce or inhibit sensory and/or motor function in the nerve are provided. In preferred embodiments the subject is a human. In some embodiments, the methods are effective to delay the onset of pain, such as nociceptive and/or neuropathic pain in the subject. In some embodiments, the subject is a human adult. In other embodiments, the subject is a human child. All the methods described can include the step of identifying a subject in need of pain relief.

In some embodiments, the methods include administering covalent-anesthetic-polymer conjugates in combination with one or more additional active agents, such as local anesthetics. The term "combination" or "combined" is used to refer to either concomitant, simultaneous, or sequential administration of two or more different agents. Therefore, the combinations can be administered either concomitantly (*e.g*., as an admixture), separately but simultaneously (*e.g., via* separate applications into the same subject), or sequentially (*e.g*., one of the compounds or agents is given first followed by the second). For example, the combination therapy can include co-administration of TTX-PGS-PEG and/or TTX-PGS with one or more additional anesthetic or other active agents separately in two different formulations, or together in the same formulation (*i.e.,* a single pharmaceutical composition including both active agents). If the two agents are administered in separate formulations, co-administration can include the simultaneous and/or sequential administration of the two agents. An appropriate time course for sequential administration can be chosen by the physician, according to factors such as the nature of a patient's illness, and the patient's condition. In certain embodiments, sequential administration includes the co-administration of the two agents within a period of hours, days, or weeks of one another. For example, in some embodiments the TTX-PGS-PEG and/or TTX-PGS is administered first, followed by the in combination with alpha-2-adrenergic agonist. In other embodiments the TTX-PGS-PEG and/or TTX-PGS is administered first, followed by the additional active agent.

### 1. Dosages

One or more covalent-anesthetic-polymer conjugates can be administered at a discrete painful site in an amount effective to produce a selective, highly-localized nerve blockade in a discrete, localized area responsible for the initiation of pain for the purpose of reducing or eliminating pain arising from a discrete locus.

In some embodiments, dosages are effective to treat or prevent pain when administered for infiltration anesthesia of one or more tissues. In some embodiments, dosages are effective to treat or prevent pain when administered for conduction or nerve block anesthesia.

The methods minimize potential adverse consequences of the anesthetic agent outside of the locus of pain. For example, PGS-PEG and/or TTX-PGS can be administered at a discrete painful site in an amount effective to produce a selective, highly-localized nerve blockade in a discrete, localized area responsible for the initiation of pain for the purpose of reducing or eliminating pain arising from a discrete locus in the subject for a period of time from about one hour to about 72 hours, according to the needs of the subject.

Pharmaceutical compositions of covalent-anesthetic-polymer conjugates for attenuating pain at a site in a human or animal in need thereof can include from 1 $\mu$g to 1.5 mg of anesthetic agents conjugated to the polymer backbone. For example, pharmaceutical compositions of covalent-anesthetic-polymer conjugates for attenuating pain at a site in a human or animal in need thereof can include from 1 $\mu$g to 5,000 mg of conjugates. The amount of conjugates that are administered will depend upon the size and type of the polymer backbone, as well as the anesthetic agent within the conjugate. In certain embodiments, the dose of anesthetic agent within the conjugate ranges from about 1 $\mu$g to about 2000 $\mu$g, from about 10 $\mu$g to about 200 $\mu$g, or from about 20 $\mu$g to about 50 $\mu$g. Typically, at least 75%, at least 80% or 90%, up to and including 100% of the total amount of anesthetic agent is released from the polymer backbone conjugates *in vivo* over the desired period of time following administration.

The disclosed formulations for prolonged local anesthesia can be administered by any means known in the art, including *via* injection to a discrete site through the skin of a human or animal; *via* implantation to a discrete site by embedding the dose into the skin, tissue, muscles, tendons, joints, or other body parts of a human or animal; by infiltration into a discrete wound, tissue surface or surgical site where the surgical wound is open.

For prolonged anesthesia in animals and humans, the body weight of the subject can be used as a guide for determining an appropriate dosage (see Table 8, below). Different clinical situations place different demands on local anesthetic safety and efficacy. Systemic safety determines the upper limit on the total dose (mg or mg/kg) of TTX or other active agents disclosed. There are small differences in total permissible dose based on the time course of uptake, vascularity, *etc.,* but overall each local anesthetic has a maximum permissible total dose.

In any localized region of the body, a sufficient local tissue concentration of local anesthetics is required to block afferent transmission. The lowest local concentration of one or more drugs in a given location sufficient to provide pain relief is called the "minimum effective concentration" (MEC).

Thus, clinical situations that require infiltration into large tissue volumes require larger total volumes of local anesthetic at or above MEC than clinical situations that involve smaller tissue volumes. If the MEC is similar in different locations, then larger tissue volumes require a larger total dose than small tissue volumes.

The dose of anesthetic within the covalent anesthetic-polymer conjugates will depend on the anesthetic being administered, as well as the site where the local anesthetic is administered. Typically, dosage units are prepared for use in a volume ranging from about 0.1 ml to about 120 ml. In certain embodiments, anesthetic agent(s) within the PGS or PGS-PEG conjugate are present in a concentration range between 0.01% (weight/volume) and 5% (w/v), and one or more alpha-2-adrenergic agonists are present in a concentration range between 0.01% (w/v) and 5% (w/v). Typically, the total systemic dose is no more than from approximately 1 mg/kg body weight, to approximately 200 mg/kg body weight in adults. For example, in embodiments where the local anesthetic is administered *via* a regional block (*e.g.,* an ankle block), the dose of anesthetic ranges from about 1 ml up to about 30 ml of a 0.5% (w/v) solution. In other embodiments a 3 mg/kg dose (maximum 200 mg) of a 2% (w/v) solution can be administered by intra-articular infiltration. In other embodiments the dose of local anesthetic can range between 0.5 ml to about 60 ml of a 0.25% to 5% (w/v) solution.

In preferred embodiments the anesthetic agent is TTX. The TTX within the conjugate can be in a concentration ranging between 0.01 mM and 100 mM, preferably between 0.1 mM and 0.3 mM, most preferably 0.21 mM. In some embodiments, the dosage of the conjugates administered in a single application is sufficient to reduce or inhibit sensory and/or motor function for a period of one hour, two hours, three hours, four hours, five hours, six hours, ten hours, 12 hours, one day, two days, three days, four days, five days, six days, one week, two weeks, three weeks, four weeks, one month, two months, six months or more than six months. In a specific embodiment, TTX-PGS-PEG is administered in an injectable formulation in an amount sufficient to reduce pain at or around the site of administration in the subject for up to 72 hours. In an exemplary method, injection of a formulation including PGS-PEG1000-TTX and/or PGS-PEG2000-TTX containing 1.0 $\mu$g of TTX into a subject is effective to produce nerve blockade in the subject for a period of time from 1 hour to 6 hours. The experimentally determined amounts based on animal data are set forth in Table 1.2, below:

**Table 1.2: Experimentally-determined dosages of conjugates**

| Conjugate | Experimental maximum safe TTX dose for rat per kg (μg/kg)[a] | Estimated safe dose for a 70 kg human (μg) |
|---|---|---|
| PGS-PEG2000-TTX | 20.0 | 1400.0 |
| PGS-PEG1000-TTX | 23.7 | 1660.0 |
| PGS-PEG200-TTX | 35.1 | 2635.7 |
| PGM-TTX[b] | 171.4 | 12000.0 |
| PGS-TTX | >342.9 | >24000.0 |

[a] Mass of rat used in experiment = 0.35kg
[b] PGM = poly(glycerol malonic acid)

### 2. Pain to be treated

Conjugates of anesthetic agents, including site 1 sodium channel blockers and/or other anesthetics, may be used to treat many conditions where the anesthetic formulations can be administered to or adjacent to the painful area, including but not limited to the treatment of acute or chronic pain, nociceptive and neuropathic pain, pre- and post-operative pain, cancer pain, pain associated with neurotransmitter dysregulation syndromes and orthopedic disorders, sports-related injuries, acute traumatic pain, nociceptive pain, and neurotransmitter-dysregulation syndromes.

#### i. Surgery

The disclosed formulations can be used to prevent or reduce pain associated with a surgical procedure. In some embodiments, dosage units include an amount of one or more site 1 sodium channel blockers conjugated to PGS or PGS-PEG, effective for the treatment or prevention of pain associated with multiple layers of a large surgical wound for a full-length open laparotomy, thoraco-abdominal incision, or flank incision; for Cesarean delivery, open hysterectomy, esophago-gastrectomy, nephrectomy, or large abdominal cancer surgeries such as colectomies; for wound infiltration for total hip replacement (hip arthroplasty) or total knee replacement (knee arthroplasty); for peripheral nerve blocks or plexus blocks (perineural injection); for infiltration (injection along the layers of a wound); for shoulder, hand or arm surgery, infiltration or ilio-inguinal/ilio-hypogastric blocks for inguinal hernia repair, penile block for hypospadias repair, femoral block for total knee replacement or anterior cruciate ligament repair, intercostal nerve blocks for open chest surgery, or femoral and sciatic nerve blocks for leg amputation or foot and ankle surgery; to provide lumbar sympathetic blockade for complex regional pain syndrome/reflex sympathetic dystrophy or vascular insufficiency of the leg or for celiac plexus blockade for pancreatitis or cancer of the pancreas; for nerve blocks (femoral and sciatic, lumbar plexus and sciatic) for the hip or knee joint for joint replacement surgery; or to provide sciatic nerve blockade of prolonged duration where rapid motor recovery is not necessary, as for a lower leg amputation.

Dosage units containing an amount of one or more site 1 sodium channel blockers conjugated to PGS, and/or PGS-PEG to provide prolonged duration of anesthesia in the eye or ocular cavity are also provided. In other embodiments, dosage units can be prepared in an amount effective to prevent, reduce or inhibit sensory and/or motor function in the cornea of the eye.

In some embodiments, dosage units can be prepared in an amount effective to prevent, reduce or inhibit sensory and/or motor function in one or more peripheral nerves.

In other embodiments, dosage units contain an amount of one or more anesthetic agents covalently conjugated to one or more polymers to prolong the delivery or release of the anesthetic agent in or around the sciatic nerve.

#### ii. Chronic pain

In some embodiments the disclosed formulations can be used in the treatment or prevention of pain for an extended period of time. The methods can include administering to the subject a formulation including one or more anesthetic agents covalently conjugated to one or more polymers for controlled or delayed release of the anesthetic agents at the site of administration over a prolonged period of time. For example, methods including administering to a subject a formulation including TTX-PEG-PGS for controlled or delayed release of the TTX at or near the site of administration over a prolonged period of time are provided. Methods including administering covalent anesthetic-polymer conjugates containing one or more site 1 sodium channel blockers and one or more other anesthetics or other agents to a subject at or near to a painful structure in an amount effective to reduce or inhibit sensory and/or motor function for a period of one hour, two

hours, three hours, four hours, five hours, six hours, ten hours, 12 hours, one day, two days, three days, four days, five days, six days, one week, two weeks, three weeks, four weeks, one month, two months, six months or more than six months are provided.

In certain embodiments, the methods can be used for the treatment or attenuation of chronic pain, for example chronic pain associated with diseases or disorders. For example, in patients suffering from chronic pain the dose of TTX that is released from the TTX-PGS and/or TTX-PGS-PEG is up to 100 μg/hour, up to 10 μg/hour, up to 1 μg/hour, up to μg/day, or up to 1 μg/week. The amount of anesthetic administered to the subject within the initial administration can calculated in view of the desired hourly or daily dose. The total dosage and rate of release can be calculated in view of the serum half-life of the anesthetic, such that the serum concentration never exceeds that at which toxicity and/or side effects occur. Typically, the total dosage is administered at one time at or near the immediate site of the disease or disorder or to the area surrounding the painful structure.

**IV. Formulations and Methods of Making**

Animal and human studies are required to determine effective dosages and volumes for treatment of humans. For example, the rank order of potencies of site 1 sodium channel blockers from *in vitro* physiology experiments did not predict the rank order of potency of those compounds *in vivo* (Kohane, et al., Reg. Anesth. Pain Med., 25: 52-9 (2000)).

Different clinical situations place different demands on local anesthetic safety and efficacy. Systemic safety determines the upper limit on the total dose (mg or mg/kg) of TTX, or other local anesthetics. There are small differences in total permissible dose based on the time course of uptake, vascularity, *etc.,* but overall each local anesthetic has a maximum permissible total dose. As described herein, inclusion of TTX as a covalent conjugate to a PGS or PGS-PEG polymer backbone enables delayed release of TTX *via* hydrolysis of ester linkages *in vivo.* Delayed release enables prolonged nerve blockade, enhanced efficacy and systemic safety, permitting larger doses or larger

In some clinical settings, it is important not to give large volumes (*i.e.*, greater than 20 mls) of local anesthetic, in order to prevent spread of numbness or weakness to other locations in the body where these effects are undesirable or even dangerous. The described covalent anesthetic-polymer conjugates can be administered in a desired volume, for example, from 1 ml to 500 ml, according to the dose, release rate, and other parameters desired by the recipient.

As described in the Examples (below), Poly(triol dicarboxylic acid)-co-poly(ethylene glycol) (TDP) polymers were synthesized by the formation of ester bonds between the carboxyl groups of the dicarboxylid acid and the hydroxyl groups of the triol and PEG via Steglich esterification reaction. TDP polymers were degradable via the hydrolysis of ester bonds, and showed a good biocompatibility with minimal cytotoxicity. A family of TDP polymers with various $f_{phil}$ were synthesized. Both surface and bulk characterization indicate that the $f_{phil}$ of the polymers determined the rate of the hydrolysis of ester bonds of the polymer network. TDP polymers have multiple active ending groups (hydroxyl and carboxyl groups), with which drugs can covalently conjugate. These characteristics suggest TDP polymers have the potential to work as a universal platform for controlled release of a broad range of therapeutic drugs

Covalent conjugation of TTX with TDP polymer was achieved by the formation of ester bonds between the carboxyl groups of the TDP polymer and the hydroxyl groups of the TTX via Steglich esterification reaction. The Steglich esterification synthesis was performed at room temperature to avoid drug degradation. The synthesized TDP-TTX conjugates gradually degraded into smaller polymer-TTX fragments, and ultimately degraded into TTX in its native form via the hydrolysis of ester bonds to achieve a controlled release of TTX. The release rate of TTX was inversely proportional to the $f_{phil}$ of TDP polymers. With the $f_{phil}$ below 37.8%, TDP-TTX conjugates could achieve an extended *in vitro* TTX release over a one-month period.

**A. Methods of Making Covalent Polymer-Anesthetic Conjugates**

Typically, the synthesis of poly(triol dicarboxylic acid)-co-poly(ethylene glycol) (TDP) polymers is performed in a three step reaction. The reaction is typically carried out under conditions that minimize damage or denaturation of the anesthetic.

In an exemplary method, synthesis of PGS-PEG-TTX is performed in a three step reaction at room temperature to avoid drug denaturation. The first step is the Steglich esterification of PEG and sebacic acid, which results in the formation of a linear pre-polymer chain without any crosslinking. A schematic representation of the reaction is depicted in Figures 1A-1B. The hydrophilicity of the final copolymers is tuned by the addition of PEG. Exemplary methods use different molecular weights of PEG, such as 200 Da, 1,000 Da, and 2,000 Da.

The second step is the addition of glycerol, resulting in a block copolymer of PGS-PEG. As depicted in the Examples, for PGS-PEG copolymer synthesis, the molar ratio between the -COOH group and -OH group was 8:7, the extra -COOH groups were used for the drug conjugation (Tables 1, and 2).

In the third step, PGS-PEG-TTX conjugates are obtained via Steglich esterification between the remaining -COOH groups of PGS-PEG copolymers and the - OH groups of TTX (Figures 1A, 1B).

### B. Methods of Making Syringe-Injectable Formulations

In some embodiments, the methods include making syringe-injectable formulations. In an exemplary embodiment, PGS-PEG-TTX and/or PGS-TTX conjugates are fully dissolved in DCM, followed by addition of PEG200. Since PEG200 is well miscible with DCM, after vortex mixture, a uniform solution is achieved. DCM is gradually removed by rotary evaporation and lyophilization. Preferably, a uniform solution containing PGS-PEG-TTX and/or PGS-TTX conjugates and PEG200 is formed.

The present invention will be further understood by reference to the following non-limiting examples.

## Examples

### Example 1: Conjugates of Anesthetic with Amphiphilic Polymers and PEG

An amphiphilic, biodegradable conjugate of poly(glycerol sebacate) (PGS), PEG and TTX was designed, produced and assayed according to the following methods.

#### Methods

***Materials***

Sebacic acid (99%), Poly(ethylene glycol) (PEG, 200, 1000, 2000 kDa), N,N'-Diisopropylcarbodiimide (DIC, 99%), 4-dimethylaminopyridine (DMAP, 99%), anhydrous N,N-dimethylformamide (DMF, 99.8%), anhydrous dimethyl sulfoxide (DMSO, 99.9%), anhydrous dichloromethane (DCM, 99.8%), Glycerol (99%), dexamethasone (98%), fluorescein isothiocyanate isomer I (FITC, 90%), phosphate buffer saline (PBS, pH 7.4, 0.15M, 138mM NaCl, 2.7 mM KCl), chloroform-d (100%, 99.96 atom % D), hexamethylene diisocyanate (99.0%), and dibutyltin dilaurate (95.0%) were purchased from Sigma-Aldrich Inc. (St. Louis, MO). Cyanine5.5 carboxylic acid (Cy5.5, 95%) was purchased from Lumiprobe Corporation (Hallandale Beach, FL). Tetrodotoxin (TTX) was obtained from Abcam plc (Cambridge, MA); Tetrodotoxin ELISA kits were purchased from Reagen LLC (Moorestown, NJ).

***Synthesis of TDP polymers.***

The poly(triol dicarboxylic acid)-co-poly(ethylene glycol) (TDP) polymers were synthesized via a Steglich esterification, using N,N'-Diisopropylcarbodiimide (DIC) as a coupling reagent and 4-dimethylaminopyridine (DMAP) as a catalyst. Briefly, dry PEG (0.005 mol) and sebacic acid (2.02 g, 0.01 mol) were added to a round bottom flask and any remaining water was evaporated before putting the flask under nitrogen. After adding anhydrous N,N-dimethylformamide (DMF, 8 mL ) and anhydrous dichloromethane (DCM, 4 mL ), and sonicating the mixture for several minutes, DIC (4.336 mL , 0.028 mol) and DMAP (0.489 g, 0.004 mol) were added. The mixture was left at room temperature for 24 h. Glycerol (184 μl, 0.0025 mol) was added and the mixture was left at room temperature for 24 h. After reaction, DCM in the reaction mixture was removed by rotary evaporation, and then the residue was washed with 30 mL of DI water by 2 times and 30 mL of 10% ethanol by 2 times. Polymers were precipitated from water and centrifuged at 20000 rpm for 5 min. Upon drying, TDP polymers was obtained in 88-96% yield. The dried TDP polymers were stored in desiccator until further use.

***Synthesis of TDP-drug conjugates***

The TDP-drug conjugates were synthesized via the similar Steglich esterification. Briefly, dry PEG (0.005 mol) and sebacic acid (2.02 g, 0.01 mol) were added to a round bottom flask and any remaining water was evaporated before putting the flask under nitrogen. After adding anhydrous N,N-dimethylformamide (DMF, 8 mL ) and anhydrous dichloromethane (DCM, 4 mL ), and sonicating the mixture for several minutes, DIC (4.336 mL, 0.028 mol) and DMAP (0.489 g, 0.004 mol) were added. The mixture was left at room temperature for 24 h. Glycerol (184 μl, 0.0025 mol) was added and the mixture was left at room temperature for 24 h. An anhydrous DMSO (10 mL ) solution with TTX (1 mg, 0.003 mmol), and/or dexamethasone (10 mg, 0.026 mmol), and/or FITC (1 mg, 0.003 mmol), and/or Cy5.5 (1.6 mg, 0.003 mmol) was added and the mixture was left at room temperature for 7 d. After reaction, DCM in the reaction mixture was removed by rotary evaporation. In order to eliminate unbound drug and to isolate the pure polymer conjugates, the residue was washed with 30 mL of DI water by 2 times and 30 mL of 10% ethanol by 2 times. TDP-drug conjugates were precipitated from water and centrifuged at 20000 rpm for 5 min. Upon drying, TDP-drug conjugates were obtained in 88-96% yield. The dried TDP-drug conjugates were stored in desiccator until further use.

***Synthesis of $T_gD_8$-isocyanate***

Typically, 0.240 g of $T_gD_8$ (Mn = 6011, 0.4 $\times$ $10^{-3}$ mol) was dried in a 100-mL flask under high vacuum overnight. Then, 5 mL of anhydrous DMSO was added to the flask, 1.13 g of HMDI (240 μl, 1.5 $\times$ $10^{-3}$ mol) and two drops of dibutyltin dilaurate (8 $\times 10^{-3}$ g) were added sequentially. The reaction mixture was stirred at 60 °C under a nitrogen atmosphere overnight. At the end of the reaction, the resultant polymers were precipitated from diethyl ether and further purified by redissolving in 1,2-dichloroethane followed by precipitation in a mixture of methanol and diethyl ether (5/95, v/v) to remove remaining dibutyltin dilaurate. Upon drying, $T_gD_8$-isocyanate was obtained in 80-95% yield.

***Synthesis of $T_gD_8$-TTX urethane***

Typically, 0.325g of $T_gD_8$-isocyanate was dried in a 100-mL flask under high vacuum overnight. Then, 5 mL of anhydrous DMSO was added to the flask, one drops of dibutyltin dilaurate (4 $\times$ $10^{-3}$ g) and 0.1 mg of TTX were added sequentially. The reaction mixture was stirred at 60 °C under a nitrogen atmosphere overnight. At the end of the reaction, the resultant polymers were precipitated from diethyl ether and further purified by redissolving in 1,2-dichloroethane followed by precipitation in a mixture of methanol and diethyl ether (5/95, v/v) to remove remaining dibutyltin dilaurate. Upon drying, $T_gD_8$-TTX urethane was obtained in 90-95% yield.

***Contact angle procedure***

Water contact angle measurements were conducted for the polymer film spincoated on silicon wafer substrate with a goniometer equipped with an automatic dispenser (Rame-Hart, model 500). The static sessile drop method was applied. A water volume of 1 $\mu$L was deposited on the sample surface and the contact angle was determined based on the high resolution image.

***$^1$H NMR measurements***

Polymer and polymer-drug conjugates were analyzed using Nuclear magnetic resonance ($^1$H NMR) spectroscopy (Varian 400 MHz equipped with 5 mm AutoX OneProbe and Varian 7600 autosampler). Polymers were dissolved in $CDCl_3$ and the spectra were recorded at 400 MHz. The chemical shifts (δ, in ppm) for the peaks corresponding to the hydrogens in italics in the following list of polymers are provided. s/d/m indicate the shape of a peak (*i.e.,* singlet, doublet, triplet). $^1$H NMR ($T_gD_8$) (400 MHz, CDCl3) δ/ppm: 1.30 (2H, m, -C*H*$_2$-), 1.62 (2H, d, -C*H*$_2$CH$_2$O(CO)-), 2.35 (2H, m, -C*H*$_2$O(CO)-), 3.50-3.85 (2H, m, OHC*H*$_2$CHO-), 3.94 (1H, m, -OCH$_2$C*H*OH), 4.05-4.35 (2H, m, -OC*H*$_2$CHO-), 5.09 (1H, s, OHCH2CHO-), 5.26 (1H, s, -OCH$_2$C*H*O-). $^1$H NMR ($T_gD_8P_{1k}$) (400 MHz, CDCl3) δ/ppm: 1.30 (2H, m, -C*H*$_2$-), 1.62 (2H, d, -C*H*$_2$CH$_2$O(CO)-), 2.35 (2H, m, -C*H*$_2$O(CO)-), 3.64 (2H, m, -OC*H*$_2$-), 3.94 (1H, m, - OCH$_2$C*H*OH), 4.05-4.35 (2H, m, -OC*H*$_2$CHO-), 5.09 (1H, s, OHCH2C*H*O-), 5.26 (1H, s, -OCH$_2$C*H*O-). The $^1$H NMR spectrum of all TDP polymers is shown in Figure 1a with key structural elements assigned. Some peaks could not be assigned due to signal overlapping.

***FTIR measurements***

Fourier Transform Infrared (FTIR) spectra of the samples were recorded using Alpha Bruker spectrometer. The average value of 48 scans at 4 $cm^{-1}$ resolutions were collected for each sample.

***Molecular weight measurement***

The molecular weight was measured using a Waters gel permeation chromatograph (GPC) model 440. THF was used as the mobile phase at a flow rate of 1.0 mL /min. Molecular weight and polydispersity of macromonomers and copolymers were calibrated with polystyrene standards.

***In vitro degradation of polymers***

Mass loss studies were performed by placing polymers into a Slide-A-Lyzer MINI dialysis device (Thermo Scientific, Tewksbury, MA) with a 10,000 MW cut-off, further dialyzed with 14 mL of PBS and incubated at 37°C on a platform shaker (New Brunswick Innova 40, 60 rpm). At each time point, the dialysis solution was exchanged with fresh, pre-warmed PBS. 14 mL of the dialysis solution was frozen, lyophilized, and the residue mass was weighted for mass loss analysis. All experiments were done in triplicates.

***Cell culture***

Cell culture of C2C12 mouse myoblasts (American Type Culture Collection (ATCC) CRL-1772) and PC12 rat adrenal gland pheochromocytoma cells (ATCC, CRL-1772) was performed as reported[2]. In brief, C2C12 cells were cultured in DMEM with 20% FBS and 1% Penicillin Streptomycin (Invitrogen). Cells were seeded onto a 24-well plate at 50,000 cells/mL and incubated for 10-14 days in DMEM with 2% horse serum and 1% Penicillin Streptomycin to differentiate into myotubules. PC12 cells were grown in DMEM with 12.5% horse serum, 2.5% FBS and 1% Penicillin Streptomycin. Cells were seeded onto a 24 well-plate, and 50 ng/mL nerve growth factor was added 24 h after seeding (Invitrogen).

***Cell viability***

Cells ($1\times10^4$/well) were incubated with various concentrations of polymer-TTX conjugates for 24h. After incubation, cells were washed up to 5 times with PBS to remove polymers and cell viability was determined by MTT. Briefly, culture supernatants from control and polymer-containing wells were collected and cells incubated with MTT (0.5 mg/mL; 3h). The formazon was dissolved in 200 μl DMSO and optical density measured at 550 nm. The absorbance of control wells was assumed 100% and cell viability of treated wells was determined with respect to control wells.

***Equilibrium solubility method[1]***

Solubility studies of TTX were determined by equilibrating excess amounts of TTX in organic solvents. Assays were performed in plastic flasks with a capacity of 2 mL. In each flask 1 mL of organic solvent and 1 mg TTX were added. The TTX amount was sufficient to saturate each solvent, which was characterized by depositing of undissolved TTX. An incubator shaker was used to keep samples at 25°C during the test with agitation of 150 rpm for 72 hours (until the samples achieved the equilibrium condition). After this period, samples were immediately filtered through a 0.45 μm pore-size disposable capsule filter (Dezani, et al. Brazilian Journal of Pharmaceutical Sciences 49, 853-863 (2013)).

***TTX solubility in DMSO and DMF***

50 μl of filtrates were diluted with 450 μl PBS. The TTX concentration in the mixture was measured with a TTX Elisa kit. For the quantification process of TTX, a TTX ELISA Kit was used at maximum absorbance wavelengths for each solvent and the solubility values were calculated using calibration curves determined for each substance. Identical ratios of DMSO or DMF were added into standard solutions in a TTX Elisa Kit, and the standard curve was measured. The concentration is considered the saturation or equilibrium solubility of TTX.

***TTX solubility in DCM***

1 ml of the filtrates was transferred into a round-bottom flask and DCM was removed via rotary evaporation. 0.1 ml of citric buffer was added into the round-bottom flask to dissolve any TTX. 50 μl of the solution was diluted with 450 μl PBS. The TTX concentration in the mixture was measured with a TTX Elisa kit.

***Degree of drug binding measurement.***

After the esterification reaction was complete, DCM in the reaction mixture was removed by rotary evaporation, and then the reaction mixture was washed with 30 mL of DI water. Polymer-drug conjugates were centrifuged at 20000 rpm for 5 min, the supernatant was collected as supernatant #1. The polymer-drug conjugates were washed with 40 mL of DI water and centrifuged again. The supernatant was collected as supernatant #2. The TTX concentration of the collected supernatants was measured by ELISA. Dexamethasone concentration of the collected supernatants was determined by HPLC.

The degree of drug bonding was calculated as follows:

$$Degree\ of\ drug\ binding = \frac{Drug_{feed} - Drug_{unbound}}{Drug_{feed}} \times 100\%$$

***In vitro drug release***

Drug release and mass loss studies were performed by placing polymer-drug conjugates into a Slide-A-Lyzer

MINI dialysis device (Thermo Scientific, Tewksbury, MA) with a 10,000 MW cut-off, further dialyzed with 14 mL PBS and incubated at 37°C on a platform shaker (New Brunswick Innova 40, 60 rpm). At each time point, the dialysis solution was exchanged with fresh, pre-warmed PBS. 0.5 mL of the dialysis solution was saved for drug analysis. The concentration of TTX was quantified by ELISA. Dexamethasone concentration was determined by HPLC (Agilent 1260 Infinity, Agilent Co., Palo Alto, CA, USA) using a C18 column (Poroshell 120 EC-C18, 4.6 x 100 mm, i.d. 2.7 μm, Phenomenex, Torrance, CA, USA) and mobile phase acetonitrile/water (70:30) and a flow rate of 0.5 mL/min. Dexamethasone was detected by UV absorbance at $\lambda$ = 254 nm.

***LC-MS instrumentation and conditions***

Analysis was performed on a Waters® Xevo™ TQ MS ACQUITY UPLC® instrument. Chromatographic separation was achieved using a Kinetex Hilic column (100 × 2.1 mm, 100 Å, 2.6-μm particles; Phenomenex) with an inline filter assembly (Waters) and 0.05% (v/v) formic acid in water as mobile phase A and 0.05% (v/v) formic acid in methanol as mobile phase B. Gradient elution with 10%-30% B in 0-2.2 min, followed by a ramp to 95% B (2.2-2.5 min) and equilibration to 10% B (2.5-3.0 min), was used. The mobile phase flow rate was 500 μl/min, the column temperature was 25 °C, and the sample manager temperature was 10 °C. The injection volume was 5 μl; the injection-to-injection time was 4 min.

***Solvent evaporation method***

To prepare a syringe-injectable TDP-TTX/PEG200 formulation, a predetermined amount of TDP-TTX conjugates were fully dissolved in an excessive amount of DCM, followed by addition of a predetermined amount of PEG200. The resulting mixture was vortexed for 1 minute to obtain a uniform solution. DCM was evaporated via rotary evaporation, followed by vacuuming at room temperature overnight.

***Rheological test***

The rheological properties of the TDP-TTX/PEG200 formulations were monitored using an AR2000 rheometer (TA instruments, United States) equipped with a temperature controller. Parallel plate with 20 mm diameter was used for all the tests. The gap distance between the plates was 0.3 mm. Frequency sweeps ranging from 0.1 to 100 rad/s were conducted at room temperature. A constant 0.1 Pa stress was used.

***Animal studies***

Animal studies were conducted following protocols approved by the Boston Children's Hospital Animal Care and Use Committee in accordance with the guidelines of the International Association for the Study of Pain. Adult male Sprague-Dawley rats (Charles River Laboratories) weighing 350-400 g were housed in groups under a 12-h/12-h light/dark cycle with lights on at 6:00 AM.

After being anesthetized with isoflurane-oxygen, injections were performed at the left sciatic nerve. The animals were injected with drug/carriers using a 23-G needle. The needle was introduced postero-medial to the greater trochanter, pointing in the anteromedial direction, and upon contact with bone the formulations were injected onto the sciatic nerve.

Testing of nerve blockade was performed at a distal site in dermatomes innervated by the sciatic nerve (i.e. the sole of the left foot), while the right leg (uninjected) served as an untreated control that would demonstrate evidence of contralateral block related to systemic side effects. To assess sensory nerve blockade, hind paws were exposed in sequence (left then right) to a 56°C hot plate, and thermal latency was measured. Thermal latency was defined as the time the animal allowed its paw to remain on the hotplate. A thermal latency of 2 seconds indicated no nerve blockade (baseline), and a thermal latency of 12 seconds indicated deep nerve blockade. A successful nerve blockade was defined as a thermal latency above 7 seconds. Hind paws were removed from the hotplate after 12 seconds to prevent thermal injury.

Motor nerve block was assessed by a weight-bearing test to determine the motor strength of the rat's hindpaw. In brief, the rat was positioned with one hindpaw on a digital balance and was allowed to bear its own weight. The maximum weight that the rat could bear without the ankle touching the balance was recorded, and motor block was considered achieved when the motor strength was less than half-maximal, as described previously.

Durations of sensory block were calculated by the time required for thermal latency to return to 7 s, with 2 s as the baseline and 12 s as complete sensory block. The duration of motor block was defined as the time it took for the weight bearing to return halfway to normal from the maximal block.

### *Confocal imaging*

Under isoflurane-oxygen anesthesia, rats were injected with 0.5 mL of test formulation (25 mg of FITC-$T_gD_8$ conjugates in PEG200, 0.25 mg of fluorescein sodium in PEG200, 0.25 mg of fluorescein sodium in PBS), and euthanized at predetermined intervals after sciatic nerve injection. Sciatic nerves together with surrounding tissues were harvested and embedded into OCT compound, then frozen and stored at -20°C. Sections (10 μm) were prepared using a cryostat microtome and mounted onto glass slides. Afterwards, slides were fixed with pre-cooled 4% paraformaldehyde for 20 min at room temperature, washed in PBS buffer (pH 7.4) by 3 times. Finally, slides were mounted with ProLong Gold Antifade Mountant with DAPI (Thermo Fisher Scientific, Waltham, MA) and coverslips. All imaging was performed using a Zeiss LSM 710 multiphoton confocal microscopy.

### *IVIS imaging*

Under isoflurane-oxygen anesthesia, rats were shaved and injected with 0.5 mL of test formulation (25 mg of Cy5.5-$T_gD_8$ conjugates in PEG200). The in vivo fluorescence images were captured and the fluorescence intensity was evaluated at different time points post-injection using IVIS Spectrum (PerkinElmer, Inc., USA). Whole body animal images were recorded non-invasively. For ex-vivo tissue distribution studies, rats were euthanized at 1 day after the injection. To record the images, animals were anesthetized with isoflurane prior to each measurement and allowed to recover after the measurement. The 675 nm excitation filter and the 700 nm emission filter were used for the imaging.

### *Tissue harvesting and histology*

Rats were sacrificed at 4 and 14 days after the injection (these time points are useful in evaluating both inflammation and myotoxicity), and the sciatic nerve was removed together with surrounding tissues. The dissector was blinded to which solution each rat had been injected with. Nerves and surrounding tissues were placed in 10% formalin and processed for histology (hematoxylin-eosin stained slides) using standard techniques. Slides were analyzed by an observer (RP) blinded to the nature of individual samples. The primary morphological indicator of myotoxicity in this study was the presence of regenerating fibers (small basophilic fibers with central nuclei containing prominent nucleoli), with only occasional myofibers demonstrating ongoing degenerative changes.

Muscle samples were scored for inflammation (0-4 points) and myotoxicity (0-6 points) (Hirata, Y. in Pure and Applied Chemistry Vol. 50 979 (1978)). The inflammation score was a subjective assessment of severity (0: no inflammation, 1: peripheral inflammation, 2: deep inflammation, 3: muscular hemifascicular inflammation, 4: muscular holofascicular inflammation). The myotoxicity score reflected two characteristic features of local anesthetic myotoxicity: nuclear internalization and regeneration. Nuclear internalization is characterized by myocytes normal in size and chromicity, but with nuclei located away from their usual location at the periphery of the cell (Gewert, B., Plassmann, M. M. & MacLeod, M. Pathways for degradation of plastic polymers floating in the marine environment. Environmental Science: Processes & Impacts 17, 1513-1521 (2015)). Regeneration is characterized by shrunken myocytes with basophilic cytoplasm. Scoring was as follows: 0. normal; 1. perifascicular internalization; 2. deep internalization (>5 cell layers), 3. perifascicular regeneration, 4. deep regeneration, 5. hemifascicular regeneration, 6. holofascicular regeneration. The grade for a sample represents the worst area (most severe damage) present on the slide.

***Statistics.*** Data are presented as means ± SDs (n = 4 in release kinetics, cell work, and neurobehavioral studies). To take multiple comparisons into account, all statistical comparisons were done with the Tukey-Kramer test, using Origin software. $P < 0.05$ was considered to denote statistical significance.

## Results

### *Synthesis of TDP polymers*

The synthesis of TDP polymers was performed via Steglich esterification at room temperature (Figure 1A) of a triol, dicarboxylic acid and PEG. The hydrophilicity of the polymers could be modified by using a hydrophilic (glycerol) or hydrophobic (PCL triol) triol, altering the number of carbons in the aliphatic chain of dicarboxylic acid (1, 5, or 8), and varying the molecular weight of PEG. Here the nomenclature of synthesized TDP polymers will be $T_xD_yP_z$, where "x" represents the type of triol (g is glycerol, c is PCL triol), "y" represents the number of carbons in the aliphatic chain of dicarboxylic acid, and "z" represents the molecular weight of PEG (200, 1000, 2000 kDa) (Table 1). The presence of glycerol and PEG within TDP polymers is intended to increase the hydrophilic fraction ($f_{phil}$) of the polymers, defined as the weight percentage of glycerol and PEG within the TDP polymer.

**Table 1.** Characterization of TDP polymers

| Name[a] | $f_{phil}$ (%)[b] | Dicarboxylic acid | Triol | PEG | Mn[c] | Mw[c] | PDI[c] |
|---|---|---|---|---|---|---|---|
| $T_gD_8P_{2000}$ | 83.5 | sebacic acid | glycerol | $PEG_{2000}$ | 6643 | 11234 | 1.691 |
| $T_gD_8P_{1000}$ | 72.1 | sebacic acid | glycerol | $PEG_{1000}$ | 5578 | 7672 | 1.375 |
| $T_gD_8P_{200}$ | 37.8 | sebacic acid | glycerol | $PEG_{200}$ | 4470 | 7081 | 1.584 |
| $T_gD_1$ | 34.0 | malonic acid | glycerol | - | 4567 | 11628 | 2.546 |
| $T_gD_5$ | 28.9 | glutaric acid | glycerol | - | 5962 | 16595 | 2.783 |
| $T_gD_8$ | 21.0 | sebacic acid | glycerol | - | 6011 | 16564 | 2.756 |
| $T_cD_8$ | 0 | sebacic acid | PCL triol[d] | - | 6126 | 17073 | 2.787 |

[a] The polymer names are abbreviated or simplified as described in the main text.
[b] Hydrophilic fraction of the polymers ($f_{phil}$): weight percentage of PEG and glycerol within the polymer.
[c] As determined by GPC.
[d] PCL triol = polycaprolactone triol.

***Characterization of TDP polymers***

The structures of TDP polymers were investigated by $^1H$ NMR spectroscopy. In the $^1H$ NMR of TDP polymers, the methylene peaks of dicarboxylic acid were detected at 1.30, 1.62 and 2.35 ppm, and methylene peaks of triol were detected between 4.05-4.35 ppm. In $T_gD_8P_{200}$, $T_gD_8P_{1000}$, and $T_gD_8P_{2000}$, an additional methylene peak between 3.45-3.60 ppm was observed, indicating the presence of a PEG segment. The molecular weight of TDP polymers was determined by gel permeation chromatography (GPC) (Table 1). The Mn of the TDP polymers was in the range of 4,000-7,000.

The hydrophilicity of the TDP polymers was investigated by assessing both surface and bulk characteristics. The surface characteristics of polymer thin films were measured by optical tensiometry (geniometry). In this technique, the angle between the polymer surface and a tangent to a sessile water drop's surface (the contact angle) was determined; the contact angle correlates with hydrophilicity. An increase in the polymers' $f_{phil}$ correlated inversely with the contact angle (Figure 2A): as $f_{phil}$ increased from 0 to 83.5%, the contact angle of the polymer thin film decreased from 73.9$\pm$1.3 to 31.3 $\pm$2.1°. The bulk characteristics of polymer was investigated by comparing the solubility of polymers in water. The increase in $f_{phil}$ induced dissolution in water.

The *in vitro* rate of degradation of polymers increased with increasing $f_{phil}$ under physiological conditions (PBS, pH 7.4, 37°C) (Figure. 2B), presumably because a higher $f_{phil}$ would result in higher water uptake and accelerate ester bond hydrolysis. When the $f_{phil}$ was below 37.8% ($T_gD_8$ and $T_gD_8P_{200}$), polymers followed a near linear mass loss (Figure 2C). ***Cytotoxicity***

The cytotoxicity of TDP polymers was assessed in myotubes from the myoblast cell line C2C12, to assess potential myotoxicity, and the pheochromocytoma cell line PC12, which is commonly used in assays of neurotoxicity. TDP polymers were dispersed in PEG200 (50 mg/ml) and added to the cell culture and cell viability was evaluated by the MTT assay after 24 h (Figures 3A and 3B). There was no decrease in cell viability in any group in either cell line.

***Synthesis of TDP-TTX conjugates***

The synthesis of TDP-TTX conjugates was performed *via* Steglich esterification reactions, because they allow reactions to proceed at room temperature to avoid drug degradation (Figure 1B). The challenges of TTX conjugation arise from its low solubility in organic solvents; while TTX is highly polar, it is only sparingly soluble in acidified water. Since the Steglich esterification reaction has to be done in a dry non aqueous solvent, a, suitable organic solvent for TTX was required to facilitate TTX-to-conjugate bonding. Three anhydrous solvents -- DMF, DCM and DMSO -- were used for the Steglich esterification reaction. DMF is a good solvent for the dicarboxylic acid, and DCM is a good solvent for the catalyst (DMAP), coupling agent (DIC), glycerol, PEG, and the resulting polymer. DMSO was chosen as the solvent for TTX because TTX had its highest solubility in DMSO (10 $\mu$g/mL) among the tested organic solvents (Table 2). Specifically, 1 mg of TTX was added into the reaction mixture (see Methods). Due to its low solubility in DMSO, only a small amount of TTX was originally dissolved in DMSO and participated in the reaction. However, as the Steglich esterification reaction proceeded, the dissolved TTX was conjugated with TDP polymer, which broken the solution equilibrium of TTX in DMSO, and more TTX was subsequently dissolved in DMSO to participate in the reaction. To achieve a high degree of binding of TTX to TDP (mass fraction of TTX conjugated with polymer with respect to the total feed TTX), the reaction was allowed to occur at room temperature for 7 days. After reaction, the degree of binding was determined by measuring the unbound TTX in the reaction mixture by ELISA. >99.0% of the TTX was conjugated to TDP polymer (Table 3, Table 4, Table 5).

**Table 2.** TTX solubility in organic solvents

| Organic solvent | TTX solubility (μg/mL) |
|---|---|
| DMSO | 10.0 |
| DMF | 1.7 |
| DCM | 0 |

Please see Methods for description of the equilibrium solubility method to determine solubility.

**Table 3.** Characterization of TDP-TTX conjugates

| Conjugate | Dicarbox ylic acid (mmol) | Triol (mmol) | PEG (mmol) | TTX (mg) | TTX loading[a] (μg/mg) | Degree of binding [b](wt %) |
|---|---|---|---|---|---|---|
| $T_gD_8P_{2000}$-TTX | 5 | 1.25 | 2.5 | 1 | 0.16 | 99.2 |
| $T_eD_8P_{1000}$-TTX | 5 | 1.25 | 2.5 | 1 | 0.28 | 99.0 |
| $T_gD_8P_{200}$-TTX | 5 | 1.25 | 2.5 | 1 | 0.60 | 99.5 |
| $T_gD_8$-TTX | 5 | 2.9 | - | 2 | 1.60 | 99.4 |
| $T_gD_5$-TTX | 5 | 2.9 | - | 2 | 2.15 | 99.5 |
| $T_gD_1$-TTX | 5 | 2.9 | - | 2 | 2.55 | 99.4 |
| $T_cD_8$-TTX | 2.5 | 1.45 | - | 2 | 2.13 | 99.6 |

[a] As determined by ELISA.
[b] Degree of binding: mass fraction of TTX conjugated with polymer with respect to the total feed TTX.

**Table 4.** Measurement of degree of binding of TTX

| | DMSO (mL) | Water (mL) | TTX concentration (ng/mL)[b] | Unbound TTX (μg) |
|---|---|---|---|---|
| Supernatant #1[a] | 10 | 30 | 150 | 6 |
| Supernatant #2[a] | 0 | 40 | 0 | 0 |

Please see Methods for description of the measurement of degree of drug binding
[a]After steglich esterification reaction, DCM in the reaction mixture was removed by rotary evaporation, and then the reaction mixture containing 10 mL of DMSO, polymer-TTX conjugate and unbound TTX was washed with 30 mL of DI water. Polymer-TTX conjugates were centrifuged at 20000 rpm for 5 min, the supernatant containing 30 mL of DI water, 10 mL of DMSO and unbound TTX was collected as supernatant #1. The polymer-TTX conjugates were washed with 40 mL of DI water and centrifuged again. The supernatant containing 40 mL of DI water and unbound TTX was collected as supernatant #2.
[b]The TTX concentration of supernatants was measured with a TTX Elisa kit. Because of the presence of organic solvent in supernatant #1, supernatant #1 was diluted 10 fold with PBS to avoid the effect of organic solvent on the ELISA measurement.

Degree of TTX binding was calculated as follows:

$$\frac{TTX_{feed} - TTX_{unbound}}{TTX_{feed}} \times 100\% = \frac{1000\ \mu g - 6\ \mu g}{1000\ \mu g} \times 100\% = 99.4\%$$

**Table 5.** Synthesis and characterization of TDP-TTX conjugates

| Name | Amount added to reaction | | | | PEG concentration[a] (%) | Percent yield (%)[b] |
|---|---|---|---|---|---|---|
| | Dicarbox ylic acid (mmol) | Triol (mmol) | PEG (mmol) | TTX (mmol)) | | |
| $T_gD_8P_{2000}$-TTX | 5 | 1.25 | 2.5 | 0.003 | 68.4 | 88 |
| $T_gD_8P_{1000}$-TTX | 5 | 1.25 | 2.5 | 0.003 | 60.8 | 90 |
| $T_gD_8P_{200}$-TTX | 5 | 1.25 | 2.5 | 0.003 | 26.7 | 94 |
| $T_gD_8$-TTX | 5 | 2.9 | - | 0.006 | - | 96 |
| $T_gD_5$-TTX | 5 | 2.9 | - | 0.006 | - | 94 |
| $T_gD_1$-TTX | 5 | 2.9 | - | 0.006 | - | 90 |
| $T_cD_8$-TTX | 2.5 | 1.45 | - | 0.006 | - | 95 |

[a]PEG concentration was calculated according to the NMR ratio of methylene hydrogens within PEG and sebacic acid.

After reaction, the reaction mixture was washed two times with 40 mL of DI water. Polymer-TTX conjugates were collected after centrifugation at 20,000 rpm for 5 min, followed by freezing with liquid nitrogen and lyophilization. Actual yield of polymer-TTX conjugates was calculated by weighing the dried polymer-TTX conjugates and all reactants. Percent yield (%) of polymer-TTX conjugates was calculated as follows:

$$\%\ Yield = \frac{Actual\ Yield}{Theoretical\ Yield} \times 100\%$$

*Characterization of TDP-TTX conjugates*

The conjugation of TDP polymer with TTX was confirmed directly by Fourier-transform infrared spectroscopy (FTIR). The absorption peaks in the range of 3000-3600 $cm^{-1}$ are characteristic for the guanidium group of TTX and hydroxyl group of TDP polymer. TDP-TTX exhibited a stronger absorption in the range of 3,000-3,600 $cm^{-1}$ than that of unmodified TDP polymer, indicating the presence of TTX.

**Example 2: TTX-PGS/PEG provides long-duration *in vitro* drug release without cytotoxicity**

**Results**

To assess the potential of TDP-TTX conjugates to provide sustained nerve blockade, release kinetics were studied *in vitro* under physiological conditions (PBS, pH 7.4, 37°C). HPLC of release samples revealed a peak at ~ 5.0 min. Liquid chromatography-mass spectrometry (LC-MS) confirmed that the molecular weight of the molecule in that fraction corresponded to that of TTX (m/z 320.1 is $[TTX+H]^+$), confirming that TTX was released from TDP-TTX conjugates in its native form. The TTX release half-time, which is the time taken to release half the TTX loaded, was investigated. All TDP-TTX conjugates significantly increased the duration of TTX release, compared with free TTX (Figures 4A and 4B).

$f_{phil}$ of TDP polymers determined the release rate of TTX. As the $f_{phil}$ decreased from 83.5 to 0%, the TTX release half-time increased from 25±5 hours to 723±75 hours. In addition, TTX release followed a near linear profile at lower values of $f_{phil}$ ($T_gD_8$-TTX and $T_gD_8P_{200}$-TTX), suggesting the potential to release TTX at a constant rate for prolonged duration local anesthesia with minimal systemic toxicity.

To demonstrate that TTX release was dependent on the cleavage of ester bonds, TTX was covalently conjugated onto TDP via urethane bonds (Figure 5A), which can be hydrolyzed, but at a much slower rate than ester bond (Chaffin, K. A., Chen, X., McNamara, L., Bates, F. S. & Hillmyer, M. A. Polyether Urethane Hydrolytic Stability after Exposure to Deoxygenated Water. Macromolecules 47, 5220-5226, doi:10.1021/ma500904d (2014)). In *vitro,* after 28 days of incubation in physiological conditions (PBS, pH 7.4, 37°C), no ELISA detectable TTX was released from 25 mg of $T_gD_8$-TTX urethane conjugate containing 10 μg of TTX (Figure 6).

*In vitro drug release from PGS-PEG-TTX and PGS-TTX conjugates*

The *in vitro* drug release and degradation of PGS-PEG-TTX and PGS-TTX conjugates were investigated under physiological conditions (PBS, 37°C) over a period of 28 days. LC-MS analyses confirmed that TTX was released from polymer-TTX conjugates in its native form. All polymer-TTX conjugate formulations significantly increased the duration of TTX release, compared with free TTX (Figures 6B, 6C). The addition of PEG into the PGS-TTX conjugate determined the hydrophilicity of the polymer backbone, and the hydrophilicity of the polymer backbone determined the hydrolysis rate of ester bonds and the TTX release rate. Thus, the TTX release rate can be adjusted by tuning the percentage of PEG segment in the polymer-TTX conjugates. The release of TTX increased in proportion to the amount of PEG incorporated. After 28 days, PGS-PEG2000-TTX showed 99.7% TTX release, whereas PGS-PEG1000-TTX, PGS-PEG200-TTX, and PGS-TTX indicated 89.5, 62.5, and 43.7% TTX release, respectively. In the cases of the PGS-PEG200-TTX and PGS-TTX conjugates, TTX was released following a zero-order release profile, which is attributed to the degradation of PGS-based copolymers under a surface erosion mechanism. The increase in TTX release with an increase in PEG concentration can be attributed to an increase in the hydrophilicity of the copolymer backbone. Such increase in hydrophilicity results in greater water uptake, which accelerates the hydrolysis of ester bonds.

Similar trends were shown for the drug-dexamethasone, whose molecule has a hydroxyl group that allows it to be covalently conjugated onto PGS-PEG *via* the same ester bond used in the conjugation of TTX. Concentrations of dexamethasone were measured using HPLC. The drug release profiles for TTX and dexamethasone were consistent for all PGS-PEG-drug conjugates studied (see Table 6). Mass loss followed a similar trend to that of drug release for PGS-PEG-TTX conjugates (Figures 6A-6E). After 28 days, PGS-TTX had a 27.3% mass loss, whereas PGS-PEG200-TTX, PGS-PEG1000-TTX and PGS-PEG2000-TTX had a mass loss of 30.4, 70.4 and 94.7%, respectively. These results confirmed that drug release occurred due to the hydrolysis of ester bonds. The tunable sustained release of the compounds of interest for several weeks supported the conjugates' potential to provide prolonged duration local anesthesia.

The data demonstrated that any drugs containing hydroxyl or carboxyl groups could be covalently conjugated with TDP polymers via hydrolyzable ester bonds. TDP polymers have the potential to work as a universal platform for controlled release of a broad range of therapeutic drugs such as dexamethasone (Figure 5B, Table 6, Figures 6A-6E).

Table 6. Synthesis and characterization of TDP-Dex conjugates (not part of the invention)

| Conjugate | Feed amount | | | | Dex loading[b] (μg/mg) | Degree of binding (%) |
|---|---|---|---|---|---|---|
| | Sebacic acid (mmol) | Triol (mmol) | PEG (mmol) | Dex[a] (mmol) | | |
| $T_gD_8P_{2000}$-Dex | 5 | 1.25 | 2.5 | 0.025 | 1.6 | 99.1 |
| $T_gD_8P_{1000}$-Dex | 5 | 1.25 | 2.5 | 0.025 | 2.8 | 99.0 |
| $T_gD_8P_{200}$-Dex | 5 | 1.25 | 2.5 | 0.025 | 6.0 | 99.4 |
| $T_gD_8$-Dex | 5 | 2.9 | - | 0.05 | 16 | 99.4 |
| $T_gD_5$-Dex | 5 | 2.9 | - | 0.05 | 21.5 | 99.5 |
| $T_gD_1$-Dex | 5 | 2.9 | - | 0.05 | 25.5 | 99.5 |
| $T_cD_8$-Dex | 2.5 | 1.45 | - | 0.05 | 21.3 | 99.6 |

[a] Dex = dexamethasone.
[b] As determined by HPLC.

**Example 3: Fabrication of syringe-injectable formulation**

**Results**

Injectable solutions and suspensions have the potential to be injected by any route of administration into the body (Mastropietro, D., Nimroozi, R. & Omidian, H. Rheology in pharmaceutical formulations-A perspective. J Dev Drugs 2, 108 (2013)), However, although the $T_gD_8P_{2000}$ that had a high $f_{phil}$ of 83.5% could be homogeneously suspended in PBS to make an injectable formulation, other TDP polymers with a low $f_{phil}$ could not be homogeneously suspended in PBS (Figures 3A and 3B). In order to administer TDP-TTX conjugates by injection into patients, a homogeneous TDP-TTX/PEG200 formulation was made by solvent evaporation (Figure 7). In brief, TDP-TTX conjugates were dissolved in DCM to make a homogeneous solution, followed by addition of PEG200, which is miscible with DCM. DCM was removed by rotary evaporation and lyophilization, leaving a homogeneous suspension of TDP-TTX in PEG200.

The dynamic storage (G') and loss (G") moduli and complex viscosity of the TDP-TTX/PEG200 formulations were characterized at a range of angular frequencies (Figures 8A-8C). Viscosity is important when considering syringeability and injectability. At 50 mg/mL, all TDP-TTX/PEG200 formulations had a viscosity less than 10 Pa.s in the range of angular frequencies tested. G" was higher than G', indicating that the viscous component of the complex modulus dominated the materials' behaviors, *i.e.,* they behaved as liquids (Figure 8C). The viscous behavior and low viscosity of TDP-TTX/PEG200 formulations indicate that they are syringe-injectable.

The $f_{phil}$ of the polymers was inversely related to the viscosity of the TDP-TTX/PEG200 formulation (Figure 8A), presumably because decreasing hydrophilicity meant less miscibility with PEG200. The viscosity of $T_cD_8$-TTX/PEG200 was less than that of $T_gD_8$-TTX/PEG200, which can be attributed to the better miscibility of PCL-triol with PEG200 than that of glycerol.

Example 4: TDP-TTX conjugates induce sciatic nerve blockade *In vivo*

**Results**

***Effect of TTX dose on nerve blockade***

Rats (4 in each group) were injected at the left sciatic nerve with 0.5 mL of PBS or PEG200 containing free TTX or 0.5 mL of PEG200 containing TDP-TTX conjugates. They then underwent neurobehavioral testing to determine the duration of functional deficits *(i.e.,* nerve blockade) in both hindpaws. The duration of deficits on the injected (left) side reflected duration of nerve block. Deficits on the uninjected (right, contralateral) side reflected systemic TTX distribution.

***Free TTX with and without PEG200***

Groups of rats receiving sciatic nerve injections of free TTX showed dose-dependent increases in the frequency of successful nerve blocks and median duration of nerve block (Figures 9A and 9B). Low doses of TTX in PBS (1 or 2 μg; 6 or 12 μM respectively) caused no detectable nerve block or toxicity. Block from 4 μg (24 μM) of free TTX in PBS was successful in 100% of animals and produced a median duration of sensory nerve block of 1.9±1.0 hours; this is comparable to the effect of 0.5% bupivacaine - an anesthetic commonly used in the clinic. However, blocks with 4 μg

of TTX in PBS were associated with marked systemic toxicity, as evidenced by sensory deficits in the uninjected (contralateral) leg (Figure 9C, and Figures 10A-10E). Injection with 5 μg (30 μM) of free TTX in PBS caused contralateral deficits in all animals and was uniformly fatal (Figure 9D). There was no statistically significant difference between the durations of sensory and motor nerve blockade at any dose of free TTX in PBS (Figure 11).

Free TTX in 0.5 mL of PEG200 resulted in a much higher rate of successful nerve blockade and longer duration of nerve block than did TTX in PBS (Figures 9A, 9B). 1 μg (6 μM) and 3 μg (18 μM) of free TTX in PEG200 resulted in 100% blockade with a duration of 3.6±0.3 hours and 5.3±0.3 hours, respectively. PEG200 did not affect the incidence of systemic toxicity (Figures 9C, 9D). The improvement in success rate and duration of nerve block with PEG200 are consistent with the effects of chemical permeation enhancers or nanoencapsulation.

***$T_gD_8$-TTX/PEG200***

Nerve block duration was significantly prolonged by formulation in $T_gD_8$-TTX/PEG200 (Figures 9A, 9B). 1 μg (6 μM) of conjugated TTX in $T_gD_8$-TTX/PEG200 had 50% successful blocks with a median duration of 1.6±1.1 hours. Sensory block success and duration increased as the doses of conjugated TTX increased. Sensory nerve block with 80 μg (480 μM) of conjugated TTX in $T_gD_8$-TTX/PEG200 lasted 71.5±6.9 hours (Figure 9B) and no animals died or had contralateral deficits (Figures 9C, 9D, and Figures 10A-10E). Significantly, in this animal model it is not possible, due to limiting toxicity, to achieve such long nerve blocks with TTX in the absence of sustained release (Rwei, A. Y. et al. Repeatable and adjustable on-demand sciatic nerve block with phototriggerable liposomes. Proceedings of the National Academy of Sciences 112, 15719-15724, (2015)), chemical permeation enhancers (Lahaye, L. A. & Butterworth, I. V. J. F. Site-1 Sodium Channel Blockers as Local AnestheticsWill Neosaxitoxin Supplant the Need for Continuous Nerve Blocks? Anesthesiology 123, 741-742 (2015)), or drugs that enhance the effect of S1SCBs (Rai, R., Tallawi, M., Grigore, A. & Boccaccini, A. R. Synthesis, properties and biomedical applications of poly(glycerol sebacate) (PGS): A review. Progress in Polymer Science 37, 1051-1078, (2012)). Motor block was longer than sensory block at all doses of conjugated TTX. For example, motor block with 80 μg (480 μM) of conjugated TTX in $T_gD_8$-TTX/PEG200 lasted 83.5±10.5 hours (Figure 11).

The fact that low doses of TTX *(e.g.,* 1 - 3 μg) in $T_gD_8$-TTX/PEG200 had a greater rate of successful blocks than did free TTX in PBS was consistent with a CPE-like effect of the excipients in the formulation. Else one would generally expect the free drug to have a higher rate of successful blocks because the free fraction of drug is higher. To determine whether the PEG200 or $T_gD_8$ was responsible for CPE-like effects, 3 μg (18 μM) of free TTX together with (but not conjugated to) 25 mg of $T_gD_8$ polymer in 0.5 mL of PEG200 were injected at the sciatic nerve, resulting in 100% blockade with a duration of 3.5±1.1 h. This was longer than the duration of block from free TTX (p <0.05), but not than TTX in PEG200, indicating that the PEG200 and not the $T_gD_8$ was responsible for the CPE-like effect In the absence of TTX, $T_gD_8$ polymer in 0.5 mL of PEG200 did not cause nerve block (Table 7).

**Table 7.** Effect of PEG200 on nerve block with TTX (n = 4)

| Function | 3 μg of free TTX in 0.5 ml of PBS | 3 μg of free TTX in 0.5 ml of PEG200 | 3 μg of free TTX in 0.5 ml of PEG200 containing 25 mg of $T_gD_8$ | 0.5 ml of PEG200 containing 25 mg of $T_gD_8$ |
|---|---|---|---|---|
| Successful blocks (%) | 25 | 100 | 100 | 0 |
| Block duration (hours) | 1 | 5.3±0.3 | 3.5±1.1 | 0 |
| Contralateral block (%) | 25 | 0 | 50 | 0 |
| Mortality (%) | 0 | 0 | 0 | 0 |

Data for durations of nerve block are means ± SD.

To investigate the possibility that the PEG200 can work as a CPE molecular to enhance TTX flux into nerve, fluorescein sodium, a fluorescent dye with an excitation wavelength of 460 nm and emission wavelength of 515 nm, was used as a proxy for TTX because both are very hydrophilic. Animals were injected at the rat sciatic nerve with 0.25 mg of fluorescein sodium in 0.5 mL of PEG200 or PBS. One and four hours later, animals were euthanized and the sciatic nerve and surrounding tissue were harvested. Frozen sections of the tissues were produced, and fluorescent images taken. Fluorescence images of sections of sciatic nerves and surrounding tissues were captured at 1 and 4 hours after injection of 0.25 mg of fluorescein sodium in 0.5 ml of PEG or PBS.

In animals injected with fluorescein sodium in PEG200, fluorescence was observed throughout the nerve one hour after injection, but none four hours after injection. No fluorescence was observed in the nerve in animals injected with the same dose of fluorescein sodium in PBS at either time point, or in the uninjected extremity. These results demonstrated that PEG200 can act as a chemical permeation enhancer to help molecules penetrate into the nerve.

A crucial hypothesis underlying this work was that TTX bound to a polymer would be inactive, and that it would

become active when released. To test this hypothesis, 10.0 μg of conjugated TTX on $T_gD_8$ *via* urethane bonds was injected at the sciatic nerve. No sensory nerve blockade was produced in any of the animals tested. These results indicated that TTX had no biological activity when covalently conjugated onto a polymer backbone.

***$T_gD_8$-TTX/PEG200 diluted with PBS***

To exclude the CPE effect induced by PEG200 and investigate the effect of sustained release of TTX from $T_gD_8$-TTX itself on the nerve block. The free TTX/PEG200 and $T_gD_8$-TTX/PEG200 solution was diluted 4 times in PBS containing 10 wt% bovine serum albumin (BSA). BSA worked as a surfactant to avoid the precipitation of $T_gD_8$-TTX from the solution. 1 μg (6 μM) of free TTX in 0.5 ml of the diluted solution caused no detectable nerve block or toxicity, indicating the exclusion of the CPE effect as that of pure PEG200 (Table 8). 25 mg of $T_gD_8$-TTX containing 40 μg (240 μM) of TTX in 0.5 ml of the diluted solution did not cause detectable nerve block or toxicity, indicating the slow release of TTX from $T_gD_8$-TTX did not achieve the therapeutic dose of TTX to produce nerve block. These results further confirmed the CPE-like function of PEG200 in the $T_gD_8$-TTX/PEG200 formulation.

**Table 8.** Effect of PEG200/PBS solution on peripheral nerve blockade. PEG200/PBS solution was made by diluting PEG200 by 4 times in PBS containing 10 wt% bovine serum albumin (BSA).

| Function | 1 μg of free TTX in 0.5 ml of PEG/PBS | 3 μg of free TTX in 0.5 ml of PEG/PBS | 40 μg of conjugated TTX in 25 mg of $T_gD_8$ in 0.5 ml of PEG/PB S |
|---|---|---|---|
| Successful blocks (%) | 0 | 0 | 0 |
| Block duration (hours) | 0 | 0 | 0 |
| Contralateral block (%) | 0 | 0 | 0 |
| Mortality (%) | 0 | 100 | 0 |

Data for durations of nerve block are means ± SD.

**Example 5:$f_{phil}$ Effects toxicity and efficacy of conjugated TTX**

Inspired by the fact that$f_{phil}$ of TDP polymers determined the TTX release rate from TDP-TTX conjugates *in vitro,* our hypothesis is that$f_{phil}$ of TDP polymers also determines the toxicity and efficacy of a specific dose of conjugated TTX *in vivo: a* higher $f_{phil}$ induced a faster release of TTX in its native form, therefore increased the toxicity of a specific high dose of conjugated TTX, while increased the efficacy of a specific low dose of conjugated TTX.

To test our hypothesis, rats were injected at the left sciatic nerve with TDP-TTX conjugates containing two doses of conjugated TTX: 10 μg (60 μM) and 1 μg (6 μM). Effect of $f_{phil}$ on the toxicity and efficacy of the conjugated TTX was examined. An increase in the $f_{phil}$ of TDP polymer increased the systemic toxicity of the 10 μg (60 μM) of conjugated TTX, as evidenced by increase in the mortality rate (Figures 12A-12D). On the other hand, an increase in the $f_{phil}$ of TDP polymer increased the efficacy of the 1 μg (6 μM) of conjugated TTX, as evidenced by increase in the rate of successful blocks and block duration.

**Example 6: TDP polymers bio-distributed at the nerve *In vivo***

**Results**

To examine the local distribution of TDP polymer following sciatic nerve injection, animals were injected with $T_gD_8$ (in 0.5 mL PEG200) to which fluorescein isothiocyanate (a fluorescent dye with an excitation wavelength of 488 nm and emission wavelength of 519 nm) was covalently conjugated so that the dye would not be able to diffuse independently; the polymer is denoted FITC-$T_gD_8$. At predetermined time points after injection, animals were euthanized and the nerves and surrounding tissues processed for histology. Fluorescent imaging by confocal showed FITC fluorescence in the connective tissue between muscle and nerve after 24, 48, and 168 hours injection. No FITC fluorescence was observed in the un-injected extremity.

To evaluate the time course of local retention of TDP in tissue animals, were injected with $T_gD_8$ (in 0.5 mL PEG200) to which Cy5.5 (a fluorescent dye emits a near-infrared signal that is ideal for fluorescence measurements in live animals) was covalently conjugated; the polymer is denoted Cy5.5-$T_gD_8$. Fluorescent imaging was taken by an *in vivo* imaging system (IVIS) to assess the location of Cy5.5-$T_gD_8$ in tissue over time. A fluorescent signal was seen at the sciatic nerve of all animals with no detectable fluorescence elsewhere. There was decrease in fluorescence signal over 4 weeks, indicating the gradual degradation of TDP *in vivo* (Figure 13). On necropsy 24 hours after injection, visible deposits were located at the sciatic nerve; these were fluorescent, confirming that they contained TDP.

**Example 7: TDP-TTX conjugates do not illicit a Tissue Reaction**

**Results**

Animals injected with free TTX and TDP-TTX conjugates with various$f_{phil}$ were euthanized 4 and 14 days after sciatic nerve injection. Representative tissue reaction to $T_gD_8$-TTX conjugates was shown in Fig. 8. At dissection, there was some residual material detected by confocal and IVIS. The tissues did not appear edematous or discolored, and had no other gross signs of tissue injury. Sciatic nerves and surrounding tissues were sectioned and harvested for histologic evaluation. Muscle tissue was processed with hematoxylin-eosin staining, and nerve tissue was processed with toluidine blue staining.

Microscopic examination did not reveal significant myotoxicity and inflammation 4 and 14 days after injection in animals injected with free TTX and TDP-TTX. The myotoxicity and inflammation were quantified using a scoring system (Table 3) (Wang, Y., Kim, Y. M. & Langer, R. In vivo degradation characteristics of poly(glycerol sebacate). Journal of Biomedical Materials Research Part A 66A, 192-197, (2003)). There was no statistically significant difference between the scores for free TTX and TDP-TTX conjugates with various$f_{phil}$.

Since H&E-stained are relatively insensitive for identifying nerve injury, toluidine blue-stained Epon-embedded sections of the sciatic nerve in animals injected with TDP-TTX were obtained. Nerve histology and microscopic images showed normal axonal distribution and myelin structure in all injected rats. Microscopic examination of nerves at 4 and 14 days post-injection revealed normal unmyelinated fibers, with no evidence of injury, such as swelling, disintegration, or dark-staining axoplasm. In all groups, myelinated fibers were occasionally found to have deposits within their axon or circular breaks within their myelin sheath, which are common findings of normal peripheral nerve fibers.

**Table 9.** Myotoxicity and inflammation from free TTX and TDP-TTX conjugates

| Formulation | Dose of polymer (mg) | Dose of TTX (μg) | Median myotoxicity score (range) | | Median inflammation score (range) | |
|---|---|---|---|---|---|---|
| | | | Day 4 | Day 14 | Day 4 | Day 14 |
| Free TTX | - | 3[a] | 0(0-0) | 0.5(0-1) | 0.5(0-1) | 0(0-0) |
| $T_gD_8$-TTX | 50[b] | 80.0 | 0(0-0) | 0(0-0) | 0.5(0-1) | 0.5(0-1) |
| $T_gD_1$-TTX | 12.5[b] | 31.9 | 0.5(0-1) | 0(0-0) | 0.5(0-1) | 0.5(0-1) |
| $T_gD_8P_{200}$-TTX | 32.5[b] | 10.0 | 0(0-0) | 0(0-0) | 0.5(0-1) | 0(0-0) |
| $T_gD_8P_{1000}$-TTX | 25[b] | 6.9 | 0(0-0) | 0.5(0-1) | 0.5(0-1) | 0.5(0-1) |
| $T_gD_8P_{2000}$-TTX | 25[b] | 3.5 | 0.5(0-1) | 0(0-0) | 0.5(0-1) | 0.5(0-1) |

[a] 3 μg free TTX in 0.5 mL of PBS, [b] TDP-TTX conjugates were formulated in 0.5 mL of PEG200. Data are median with 25th and 75th percentiles (n = 4). Inflammation scores range: 0-4; myotoxicity scores range: 0-6 (McAlvin, J. B., Reznor, G., Shankarappa, S. A., Stefanescu, C. F. & Kohane, D. S. Local Toxicity from Local Anesthetic Polymeric Microparticles. Anesthesia and analgesia 116, 794-803, (2013); F Padera, R., Tse, J., Bellas, E. & S Kohane, D. Tetrodotoxin for prolonged local anesthesia with minimal myotoxicity. Vol. 34 (2006)).

**Example 8: PGS-PEG-TTX Conjugates provide a universal therapeutic drug platform for all alcoholic or carboxylic acid drugs**

**Results**

With an adjustable hydrophilicity, PGS-PEG copolymers can work as a universal therapeutic drug platform for all alcoholic or carboxylic acid drugs. Capsaicin, a conventional local anesthetic containing one hydroxyl group, was conjugated into PGS-PEG copolymers via ester bond (Figure 14). To produce nerve blockade in a rat model, the minimum effective dose of capsaicin is approximately 50 μg, which is much higher than that of TTX27-28. Thus, to achieve the minimum effective dose of capsaicin, a higher injection dose and a faster release rate of capsaicin than that of TTX are desired. To prove this concept, PGS-capsaicin and PGS-PEG1000-capsaicin conjugates were synthesized, and each of them has two graft densities: PGS-capsaicin and PGS-PEG1000-capsaicin have graft density of 44.4 μg/mg, and PGS-capsaicin-2 and PGS-PEG1000-capsaicin-2 have graft density of 182.9 μg/mg (Table 10).

**Table 10.** Synthesis of PGS-PEG-capsaicin conjugates (not part of the invention)

| Conjugates | Feeding amount (millimolar) | | | | Cap in conjugates (μg/mg) |
|---|---|---|---|---|---|
| | Sebacic acid | Glycerol | PEG | Cap (mg) | |
| PGS-Cap | 1.25 | 0.725 | 0 | 14.5 | 44.4 |
| PGS-Cap-2 | 1.25 | 0.725 | 0 | 58 | 182.9 |
| PGS-PEG1000-Cap | 1.25 | 0.3125 | 0.625 | 40 | 44.4 |
| PGS-PEG2000-Cap-2 | 0.625 | 0.1041 | 0.3125 | 100 | 182.9 |

Consistent with the release trend of TTX and dexamethasone, capsaicin was released much faster from PGS-PEG1000-capsaicin conjugates than that of PGS-capsaicin conjugates. After 1 day of incubation in PBS, 70.8 μg of capsaicin was released from 25 mg of PGS-PEG1000-capsaicin-2 conjugate, whereas only 5.3, 21.2 and 15.2 μg of capsaicin was released from 25 mg of PGS-capsaicin, PGS-capsaicin-2, and PGS-PEG1000-capsaicin, respectively (Figure 10). *In vivo,* injection of 25 mg of PGS-PEG1000-capsaicin-2 produced nerve blockade in all animals tested, lasting from 1 day to 27 days. Whereas, injection of 25 mg of PGS-PEG1000-capsaicin, PGS-capsaicin, and PGS-capsaicin-2 did not produce nerve blockade in all animals tested (Table 11), indicating that the minimum effective dose of capsaicin was not achieved. These results demonstrated that although PGS is the best suitable platform for TTX, PGS-PEG1000 is more suitable than PGS as a platform for the controlled release of capsaicin to produce nerve blockade.

**Table 11.** Durations of nerve blockade for PGS-PEG-Capsaicin conjugates

| Polymer-Drug conjugate | N | Dose injected (mg) | Cap (theoretical ) | Duration of nerve block (d) |
|---|---|---|---|---|
| PGS-Cap | 7 | 25.0[a] | 287.5 | 0 |
| PGS-Cap-2 | 2 | 25.0[a] | 1111.1 | 0 |
| PGS-PEG1000-Cap | 2 | 25.0[a] | 1110.3 | 0 |
| PGS-PEG1000-Cap-2 | 3 | 25.0[a] | 4572.5 | 1d, 3d, >23d |
| [a] formulated in 0.5 ml PEG200 | | | | |

PGS-PEG-TTX conjugates have the potential to be applied in large animals such as humans. The toxicity of a given amount of the preparation would be greatly reduced when used in larger animals, since the median toxic dose of local anesthetics, including TTX, scales in direct proportion to the mass (volume of distribution) of the recipient2. Since the dose to block a nerve will scale with body size, both the injection dose and release rate of TTX should be higher in larger animals. With a faster TTX release rate, PGS-PEG-TTX might be more suitable than PGS-TTX in larger animals.

## Summary

TDP polymers were synthesized by the formation of ester bonds between the carboxyl groups of the dicarboxylic acid and the hydroxyl groups of the triol and PEG via Steglich esterification reaction. TDP polymers were degradable via the hydrolysis of ester bonds, and showed a good biocompatibility with minimal cytotoxicity. A family of TDP polymers with various $f_{phil}$ were synthesized. Both surface and bulk characterization indicate that the $f_{phil}$ of the polymers determined the rate of the hydrolysis of ester bonds of the polymer network. TDP polymers have multiple active ending groups (hydroxyl and carboxyl groups), with which drugs can covalently conjugate. These characteristics suggest TDP polymers have the potential to work as a universal platform for controlled release of a broad range of therapeutic drugs.

Covalent conjugation of TTX with TDP polymer was achieved by the formation of ester bonds between the carboxyl groups of the TDP polymer and the hydroxyl groups of the TTX via Steglich esterification reaction. The Steglich esterification synthesis were performed at room temperature to avoid drug degradation. The synthesized TDP-TTX conjugates gradually degraded into smaller polymer-TTX fragments, and ultimately degraded into TTX in its native form via the hydrolysis of ester bonds to achieve a controlled release of TTX. The release rate of TTX was inversely proportional to the $f_{phil}$ of TDP polymers. With the $f_{phil}$ below 37.8%, TDP-TTX conjugates could achieve an extended *in vitro* TTX release over a one-month period.

The CPE function of PEG200 was confirmed, for the first time. PEG200 can cross the perineurial barrier to enhance drug flux into nerve, which could be attributed to the amphipathic nature of PEG200. Free TTX, with the doses from 1 to 3 μg, in 0.5 mL of PEG200 resulted in 100% blockade with a block duration up to 5.3±0.3 hours; this is three times longer than the effect of 0.5% bupivacaine - an anesthetic commonly used in the clinic. Being low toxicity and already

widely used in a variety of pharmaceutical formulations (D'souza, A. A. & Shegokar, R. Polyethylene glycol (PEG): a versatile polymer for pharmaceutical applications. Expert Opinion on Drug Delivery 13, 1257-1275, doi:10.1080/17425247.2016.1182485 (2016)), PEG200 holds a huge potential to work as a TTX delivery medium for local anesthesia in the clinic.

With a good miscibility, TDP-TTX conjugates were readily dispersed in PEG200, forming a homogeneous, syringe injectable TDP-TTX/PEG200 formulation. All TDP-TTX/PEG200 formulations prepared behaved as a liquid with a viscosity less than 10 Pa.s, suggesting the potential administration into patients through syringe-injection.

*In vivo* animal test established that TDP-TTX/PEG200 formulation, achieving an extended TTX release, could significantly broaden the therapeutic index of TTX for local anesthesia. (i) TDP-TTX/PEG200 allowed the injection of dose of TTX up to 80 μg without increasing its systemic toxicity. In addition, TDP-TTX conjugates have a prolonged retention in the epineurium tissue around the nerve lasting up to 4 weeks, which allowed to maintain the therapeutic TTX concentration for a long period to prolong the duration of local anesthesia; (ii) TDP-TTX/PEG200 formulation was able to increase the efficacy of TTX. TDP-TTX/PEG200 formulation significantly enhance the effectiveness of as low as 1 μg of TTX to produce nerve blockade. Overall, in a wide range of TTX injection doses from 1.0 to 80.0 μg, TDP-TTX/PEG200 formulation provided adjustable durations of nerve block from a couple of hours to 3 days.

TDP-TTX conjugates can be applied in large animals such as humans. The toxicity of a given amount of our preparation would be greatly reduced when used in larger animals, since the median toxic dose of local anesthetics, including TTX, scales in direct proportion to the mass (volume of distribution) of the recipient[2]. Since the dose to block a nerve will scale with body size, both the injection dose and release rate of TTX should be higher in larger animals. With a faster TTX release rate, TDP-TTX conjugates with higher $f_{phil}$ might be more suitable in larger animals.

A delivery system to broaden the therapeutic index of TTX was rationally designed with the aim to introduce TTX to clinical practice. TTX was covalently conjugated onto biodegradable and biocompatible TDP backbones through hydrolyzable ester bonds. The data demonstrated that TDP-TTX conjugates can provide tunable TTX release. TTX can be released, in its native form, via the hydrolysis of the ester bonds, and the release rate can be tunable by controlling the hydrophilicity of TDP backbones. PEG200 can work as a CPE molecular to cross the perineurial barrier and enhance drug flux into nerve. PEG200 can significantly enhance the effectiveness of TTX to produce nerve blockade. TDP-TTX/PEG200, a syringe injectable formulation incorporating the sustained TTX release from TDP-TTX conjugate with the CPE function of PEG200, has been validated to significantly broaden the therapeutic window of TTX. With a wide range of TTX injection doses from 1.0 to 80.0 μg, TDP-TTX/PEG200 formulation provided adjustable durations of nerve block from a couple of hours to 3 days in *in vivo* experiments. Furthermore, nerve blockades were associated with minimal systemic toxicity and virtually no local toxicity to the muscle and the peripheral nerve. TDP-TTX/PEG200 formulation provides a successful and safe approach for adjustable and prolonged duration local anesthesia.

## Claims

**1.** A covalent anesthetic-polymer conjugate for prolonged duration local anesthesia comprising:

(a) an anesthetic agent; and
(b) a polymer backbone;

wherein

the polymer backbone comprises one or more hydrophilic or hydrophobic polymers, and optionally one or more polyethylene oxide polymers;
the polymer backbone comprises a polymer produced from a dicarboxylic acid and a triol;
the anesthetic agent is selected from tetrodotoxin (TTX), saxitoxin (STX), dicarbamoyl saxitoxin, neosaxitoxin, and the gonyautoxins;
the anesthetic agent is covalently conjugated to the polymer backbone *via a* hydrolysable linker; and
the anesthetic agent is present in an amount effective to induce effective local nerve blockade with reduced toxicity relative to the unconjugated anesthetic agent following administration to a subject at a site in need thereof.

**2.** The conjugate of claim 1, wherein the polymer backbone comprises poly(glycerol sebacate) (PGS).

**3.** The conjugate of claim 1 or claim 2, wherein the polymer backbone further comprises one or more hydrophilic polymers having a molecular weight of from about 100 Da to about 200,000 Da, inclusive, preferably about 200 Da to about 2,000 Da, inclusive.

4. The conjugate of claim 3, wherein the one or more hydrophilic polymers comprise polyethylene glycol (PEG), preferably one or more PEGs selected from PEG monomethylether (PEGMME), PEG100, PEG200, PEG300, PEG400, PEG500, PEG600, PEG700, PEG800, PEG900, PEG1000, and PEG2000.

5. The conjugate of any one of claims 1-4, further comprising one or more glucocorticoids covalently conjugated to the polymer backbone.

6. The conjugate of claim 5, wherein the one or more glucocorticoids are selected from dexamethasone, cortisone, hydrocortisone, prednisone, beclomethasone, betamethasone, flunisolide, methyl prednisone, para methasone, prednisolone, triamcinolome, alclometasone, amcinonide, clobetasol, fludrocortisone, diflurosone diacetate, fluocinolone acetonide, fluoromethalone, flurandrenolide, halcinonide, medrysone, mometasone, and pharmaceutically acceptable salts thereof.

7. A formulation for administration *in vivo* comprising the conjugate of any one of claims 1-6, wherein the formulation preferably comprises PEG such as PEG200 as an excipient.

8. A dosage unit comprising the formulation of claim 7, wherein the dosage unit comprises an amount of anesthetic agent between 0.1 $\mu$g and 200 $\mu$g, inclusive, preferably between 1 $\mu$g and 100 $\mu$g, inclusive.

9. The formulation of claim 7 or the dosage unit of claim 8 for use in a method for providing a nerve blockade in a subject in need thereof, said method comprising administering the formulation or the dosage unit in an effective amount to provide a nerve block at the site of administration, wherein the formulation is preferably in an amount effective to delay the onset of neuropathic pain in the subject such as a human.

10. The formulation or dosage form for use according to claim 9, wherein the method provides pain relief for at least 3 days, up to a week, two weeks, three weeks or a month following administration.

11. The formulation for use according to claim 9 or claim 10, wherein the formulation is administered at or near the painful site via injection or infiltration.

12. The formulation for use according to claim 9, wherein the method is a method of treating or preventing pain at a site, the covalent anesthetic-polymer conjugate is dispersed within an aqueous solution of PEG, and the formulation is administered in an amount effective to provide effective nerve blockade at the site in need for a period of at least 72 hours, up to one month following administration.

13. A method of making the conjugate of any one of claims 1-6, comprising covalently coupling one or more anesthetic agents to one or more polymer backbones.

14. The method of claim 13, wherein the covalent coupling comprises a Steglich esterification reaction.

15. The method of claim 12, wherein the covalent coupling is carried out under conditions that do not denature the anesthetic agent, or otherwise reduce the biological activity of the anesthetic agent.

## Patentansprüche

1. Kovalentes Anästhetikum-Polymer-Konjugat für eine Lokalanästhesie mit verlängerter Dauer, umfassend:

(a) ein Anästhetikum; und
(b) ein Polymergerüst;

wobei

das Polymergerüst ein oder mehrere hydrophile oder hydrophobe Polymere umfasst, und optional ein oder mehrere Polyethylenoxidpolymere;
das Polymergerüst ein Polymer umfasst, das aus einer Dicarbonsäure und einem Triol hergestellt ist;
das Anästhetikum aus Tetrodotoxin (TTX), Saxitoxin (STX), Dicarbamoyl-Saxitoxin, Neosaxitoxin und den Gonyautoxinen ausgewählt ist;

das Anästhetikum über einen hydrolysierbaren Linker kovalent an das Polymergerüst konjugiert ist; und
das Anästhetikum in einer Menge vorhanden ist, die wirksam ist, um eine wirksame lokale Nervenblockade mit verringerter Toxizität im Vergleich zu dem unkonjugierten Anästhetikum nach Verabreichung an ein Subjekt an einer Stelle, an der dies erforderlich ist, zu induzieren.

**2.** Konjugat nach Anspruch 1, wobei das Polymergerüst Poly(glycerinsebacat) (PGS) umfasst.

**3.** Konjugat nach Anspruch 1 oder Anspruch 2, wobei das Polymergerüst weiter ein oder mehrere hydrophile Polymere mit einem Molekulargewicht von etwa 100 Da bis etwa 200.000 Da, einschließlich, vorzugsweise etwa 200 Da bis etwa 2.000 Da, einschließlich, umfasst.

**4.** Konjugat nach Anspruch 3, wobei das eine oder die mehreren hydrophilen Polymere Polyethylenglykol (PEG), vorzugsweise ein oder mehrere PEGs, ausgewählt aus PEG-Monomethylether (PEGMME), PEG100, PEG200, PEG300, PEG400, PEG500, PEG600, PEG700, PEG800, PEG900, PEG1000 und PEG2000 umfassen.

**5.** Konjugat nach einem der Ansprüche 1-4, weiter umfassend ein oder mehrere Glukokortikoide, die kovalent an das Polymergerüst konjugiert sind.

**6.** Konjugat nach Anspruch 5, wobei das eine oder die mehreren Glukokortikoide aus Dexamethason, Cortison, Hydrocortison, Prednison, Beclomethason, Betamethason, Flunisolid, Methylprednison, Paramethason, Prednisolon, Triamcinolon, Alclometason, Amcinonid, Clobetasol, Fludrocortison, Diflurosondiacetat, Fluocinolonacetonid, Fluoromethalon, Flurandrenolid, Halcinonid, Medryson, Mometason und pharmazeutisch annehmbaren Salzen dieser ausgewählt sind.

**7.** Formulierung zur Verabreichung *in vivo,* umfassend das Konjugat nach einem der Ansprüche 1-6, wobei die Formulierung vorzugsweise PEG, wie beispielsweise PEG200, als Hilfsstoff umfasst.

**8.** Dosierungseinheit, umfassend die Formulierung nach Anspruch 7, wobei die Dosierungseinheit eine Menge an Anästhetikum zwischen 0,1 $\mu$g und 200 $\mu$g, einschließlich, vorzugsweise zwischen 1 $\mu$g und 100 $\mu$g, einschließlich, umfasst.

**9.** Formulierung nach Anspruch 7 oder Dosierungseinheit nach Anspruch 8, zur Verwendung in einem Verfahren zur Bereitstellung einer Nervenblockade bei einem Subjekt, das diese benötigt, wobei das Verfahren die Verabreichung der Formulierung oder der Dosierungseinheit in einer wirksamen Menge umfasst, um eine Nervenblockade an der Verabreichungsstelle bereitzustellen, wobei die Formulierung vorzugsweise in einer Menge vorliegt, die wirksam ist, um das Auftreten neuropathischer Schmerzen bei dem Subjekt, beispielsweise einem Menschen, zu verzögern.

**10.** Formulierung oder Dosierungseinheit zur Verwendung nach Anspruch 9, wobei das Verfahren eine Schmerzlinderung für mindestens 3 Tage, bis zu einer Woche, zwei Wochen, drei Wochen oder einem Monat nach der Verabreichung bewirkt.

**11.** Formulierung zur Verwendung nach Anspruch 9 oder Anspruch 10, wobei die Formulierung an oder in der Nähe der schmerzenden Stelle durch Injektion oder Infiltration verabreicht wird.

**12.** Formulierung zur Verwendung nach Anspruch 9, wobei das Verfahren ein Verfahren zur Behandlung oder Vorbeugung von Schmerzen an einer Stelle ist, das kovalente Anästhetikum-Polymer-Konjugat in einer wässrigen Lösung von PEG dispergiert ist und die Formulierung in einer Menge verabreicht wird, die wirksam ist, um eine wirksame Nervenblockade an der Stelle, an der sie benötigt wird, für einen Zeitraum von mindestens 72 Stunden bis zu einem Monat nach der Verabreichung zu erzielen.

**13.** Verfahren zur Herstellung des Konjugats nach einem der Ansprüche 1-6, umfassend die kovalente Kopplung eines oder mehrerer Anästhetika an ein oder mehrere Polymergerüste.

**14.** Verfahren nach Anspruch 13, wobei die kovalente Kopplung eine Steglich-Veresterungsreaktion umfasst.

**15.** Verfahren nach Anspruch 12, wobei die kovalente Kopplung unter Bedingungen durchgeführt wird, die das Anästhetikum nicht denaturieren oder anderweitig die biologische Aktivität des Anästhetikums verringern.

## Revendications

1. Conjugué covalent anesthésique-polymère pour anesthésie locale à durée prolongée comprenant :

(a) un agent anesthésique ; et
(b) un squelette polymère ;

dans lequel

le squelette polymère comprend un ou plusieurs polymères hydrophiles ou hydrophobes, et facultativement un ou plusieurs polymères de poly(oxyde d'éthylène) ;
le squelette polymère comprend un polymère produit à partir d'un acide dicarboxylique et d'un triol ;
l'agent anesthésique est choisi parmi la tétrodotoxine (TTX), la saxitoxine (STX), la dicarbamoyl saxitoxine, la néosaxitoxine et les gonyautoxines ;
l'agent anesthésique est conjugué de manière covalente au squelette polymère *via* un liant hydrolysable ; et
l'agent anesthésique est présent en une quantité efficace pour induire un blocage nerveux local efficace avec une toxicité réduite par rapport à l'agent anesthésique non conjugué après administration à un sujet dans un site qui en a besoin.

2. Conjugué selon la revendication 1, dans lequel le squelette polymère comprend un poly(sébacate de glycérol) (PGS).

3. Conjugué selon la revendication 1 ou la revendication 2, dans lequel le squelette polymère comprend en outre un ou plusieurs polymères hydrophiles présentant un poids moléculaire d'environ 100 Da à environ 200 000 Da inclus, de préférence d'environ 200 Da à environ 2 000 Da, inclus.

4. Conjugué selon la revendication 3, dans lequel les un ou plusieurs polymères hydrophiles comprennent un polyéthylène-glycol (PEG), de préférence un ou plusieurs PEG choisis parmi le monométhyléther de PEG (PEGMME), le PEG100, le PEG200, le PEG300, le PEG400, le PEG500, le PEG600, le PEG700, le PEG800, le PEG900, le PEG1000, et le PEG2000.

5. Conjugué selon l'une quelconque des revendications 1 à 4, comprenant en outre un ou plusieurs glucocorticoïdes conjugués de manière covalente au squelette polymère.

6. Conjugué selon la revendication 5, dans lequel les un ou plusieurs glucocorticoïdes sont choisis parmi la dexaméthasone, la cortisone, l'hydrocortisone, la prednisone, la béclométhasone, la bétaméthasone, le flunisolide, la méthylprednisone, la para-méthasone, la prednisolone, la triamcinolome, l'alclométasone, l'amcinonide, le clobétasol, la fludrocortisone, le diacétate de diflurosone, l'acétonide de fluocinolone, la fluorométhalone, le flurandrénolide, l'halcinonide, la médrysone, la mométasone, et les sels pharmaceutiquement acceptables de ceux-ci.

7. Formulation pour administration *in vivo* comprenant le conjugué selon l'une quelconque des revendications 1 à 6, dans laquelle la formulation comprend de préférence un PEG, tel que le PEG200, en tant qu'excipient.

8. Unité de dosage comprenant la formulation selon la revendication 7, dans laquelle l'unité de dosage comprend une quantité d'agent anesthésique entre 0,1 $\mu$g et 200 $\mu$g, inclus, de préférence entre 1 $\mu$g et 100 $\mu$g, inclus.

9. Formulation selon la revendication 7 ou unité de dosage selon la revendication 8 pour une utilisation dans un procédé consistant à procurer un blocage nerveux chez un sujet en ayant besoin, ledit procédé comprenant l'administration de la formulation ou de l'unité de dosage en une quantité efficace pour effectuer un blocage nerveux au niveau du site d'administration, dans laquelle la formulation est de préférence en une quantité efficace pour retarder l'apparition d'une douleur neuropathique chez le sujet, tel qu'un humain.

10. Formulation ou forme de dosage pour une utilisation selon la revendication 9, dans laquelle le procédé procure un soulagement de la douleur pendant au moins 3 jours, jusqu'à une semaine, deux semaines, trois semaines ou un mois après l'administration.

11. Formulation pour une utilisation selon la revendication 9 ou la revendication 10, dans laquelle la formulation est administrée au niveau ou à proximité du site douloureux par injection ou infiltration.

**12.** Formulation pour une utilisation selon la revendication 9, dans laquelle le procédé est un procédé de traitement ou de prévention d'une douleur au niveau d'un site, le conjugué covalent anesthésique-polymère est dispersé dans une solution aqueuse de PEG, et la formulation est administrée en une quantité efficace pour fournir un blocage nerveux efficace au niveau du site en ayant besoin pendant une période d'au moins 72 heures, jusqu'à un mois après l'administration.

**13.** Procédé de préparation du conjugué selon l'une quelconque des revendications 1 à 6, comprenant le couplage covalent d'un ou de plusieurs agents anesthésiques à un ou plusieurs squelettes polymères.

**14.** Procédé selon la revendication 13, dans lequel le couplage covalent comprend une réaction d'estérification de Steglich.

**15.** Procédé selon la revendication 12, dans lequel le couplage covalent est réalisé dans des conditions qui ne dénaturent pas l'agent anesthésique, ou qui ne réduisent autrement l'activité biologique de l'agent anesthésique.

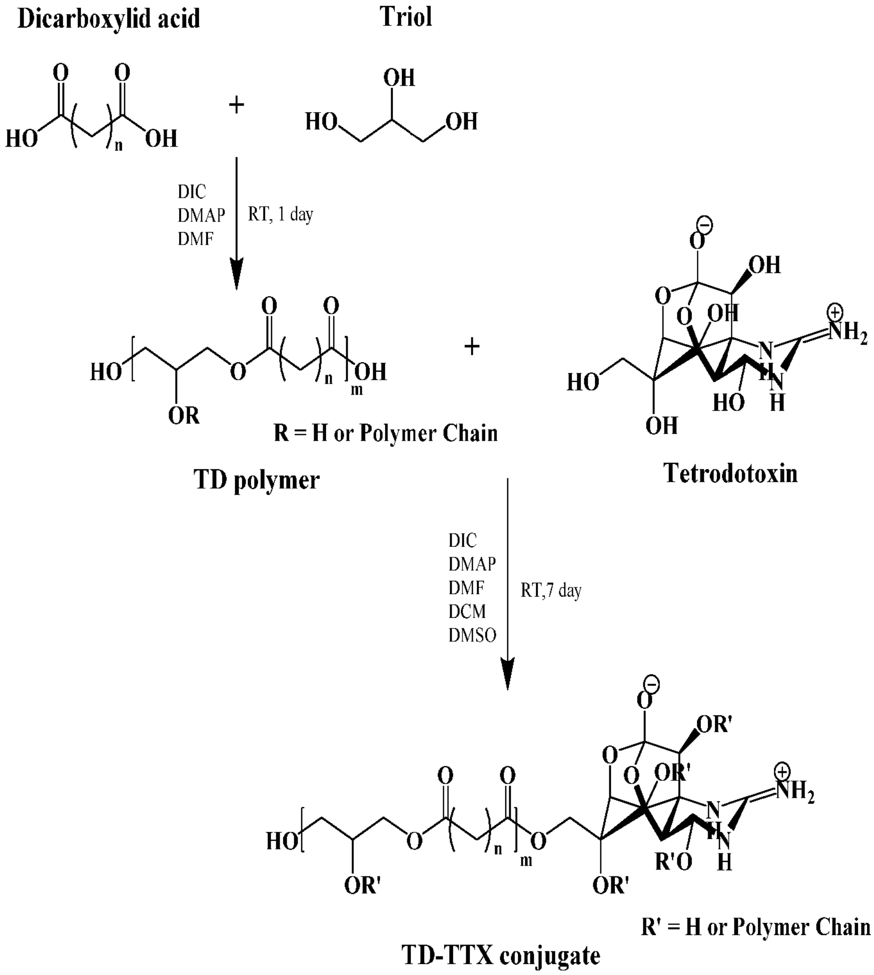
Dicarboxylid acid
Triol
DIC
DMAP
DMF
RT, 1 day
R = H or Polymer Chain
TD polymer
Tetrodotoxin
DIC
DMAP
DMF
DCM
DMSO
RT,7 day
R' = H or Polymer Chain
TD-TTX conjugate

FIG. 1A

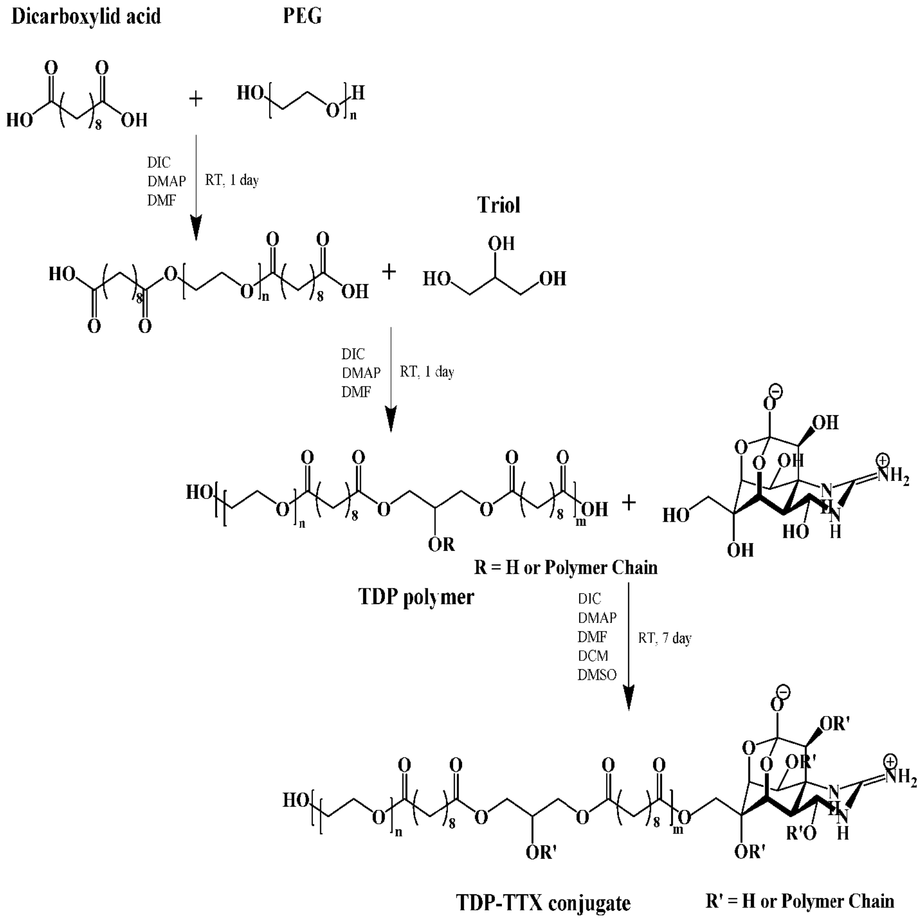
Dicarboxylid acid
PEG
DIC
DMAP
DMF
RT, 1 day
Triol
DIC
DMAP
DMF
RT, 1 day
R = H or Polymer Chain
TDP polymer
DIC
DMAP
DMF
DCM
DMSO
RT, 7 day
TDP-TTX conjugate
R' = H or Polymer Chain

FIG. 1B

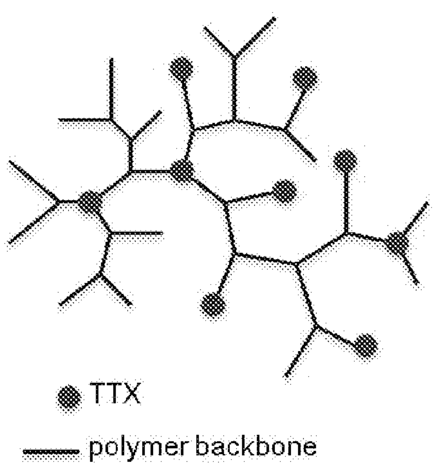
TTX
polymer backbone

FIG. 1C

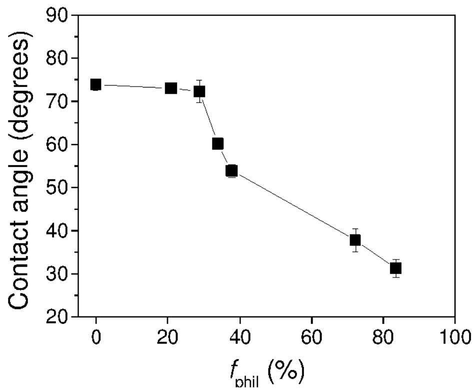


FIG. 2A

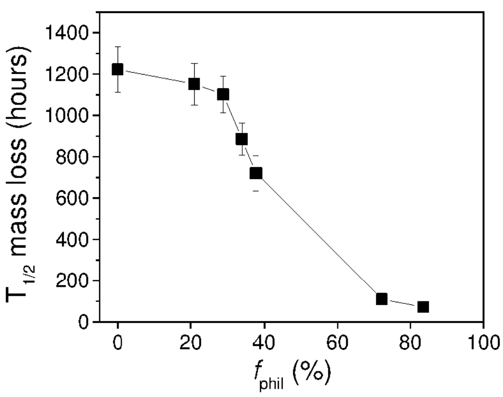


FIG. 2B

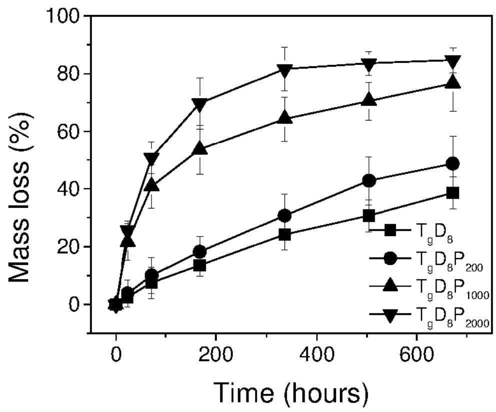


FIG. 2C

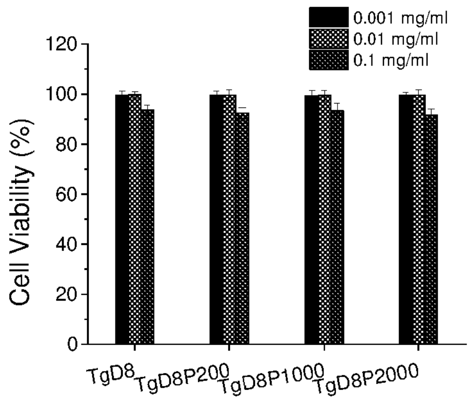


left to right: 0.001 mg/l 0.01 mg/ 0.1 mg/l

**FIG. 3A**

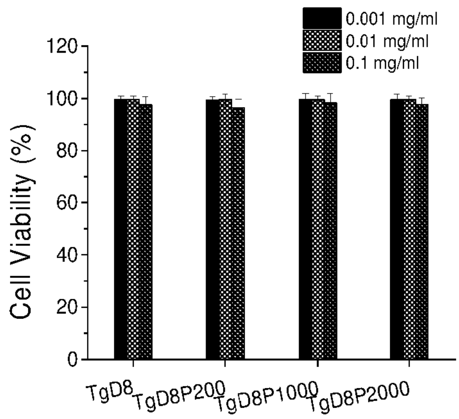


left to right: 0.001 mg/l 0.01 mg/ 0.1 mg/l

**FIG. 3B**

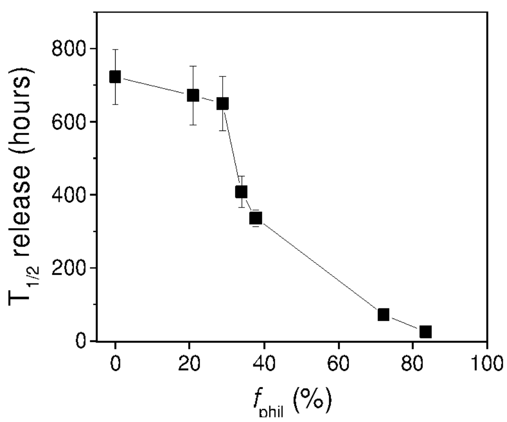


**FIG. 4A**

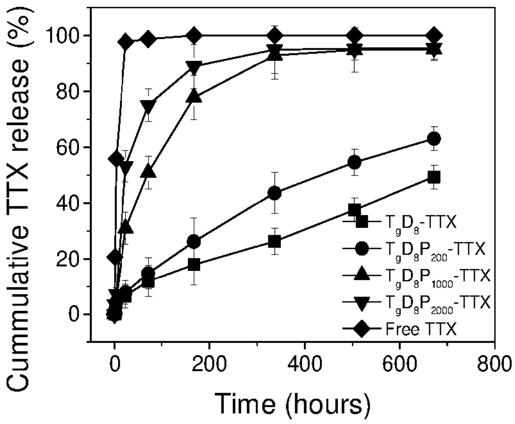
Cummulative TTX release (%)
Time (hours)
0
20
40
60
80
100
0
200
400
600
800
$T_9D_8$-TTX
$T_9D_8P_{200}$-TTX
$T_9D_8P_{1000}$-TTX
$T_9D_8P_{2000}$-TTX
Free TTX

**FIG. 4B**

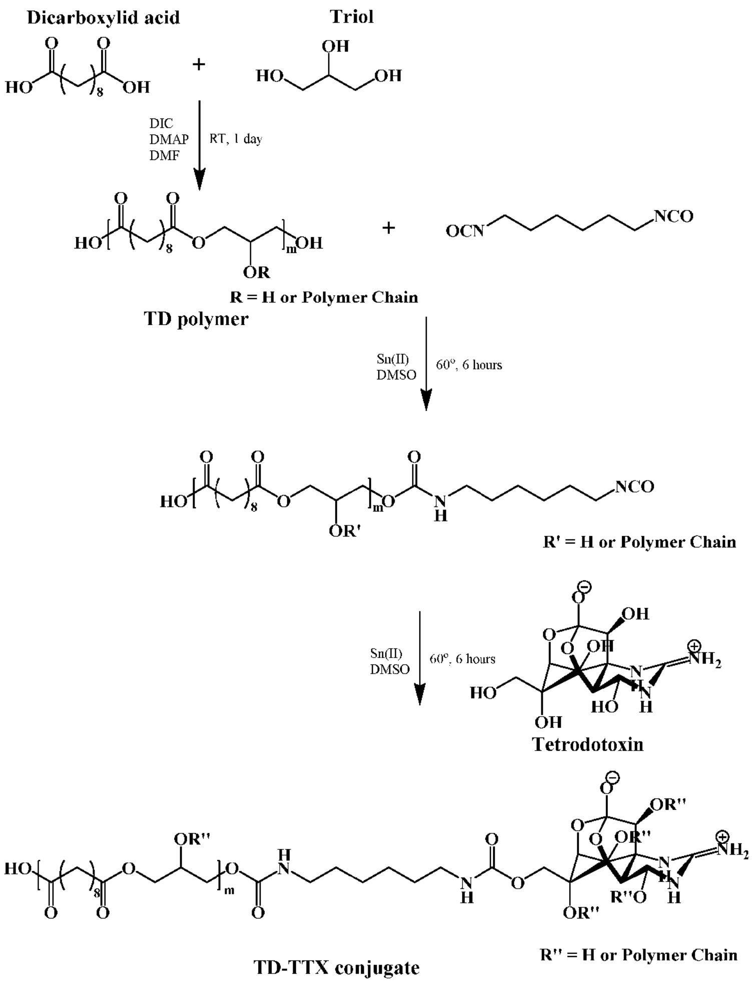
Dicarboxylid acid
Triol
DIC
DMAP
DMF
RT, 1 day
R = H or Polymer Chain
TD polymer
Sn(II)
DMSO
60°, 6 hours
R' = H or Polymer Chain
Sn(II)
DMSO
60°, 6 hours
Tetrodotoxin
R'' = H or Polymer Chain
TD-TTX conjugate

**FIG. 5A**

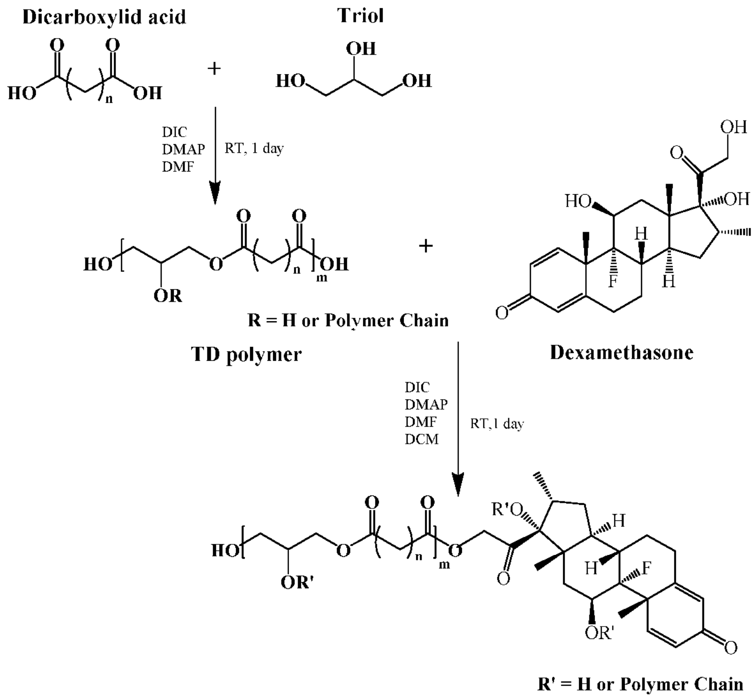


TD-Dex conjugate

FIG. 5B

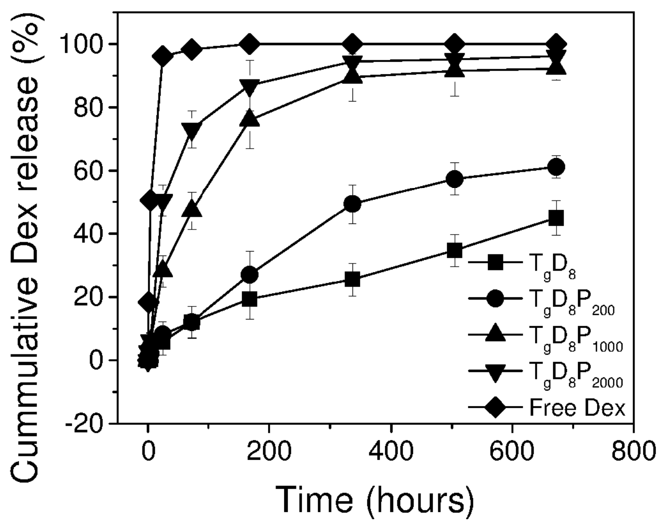


FIG. 6A

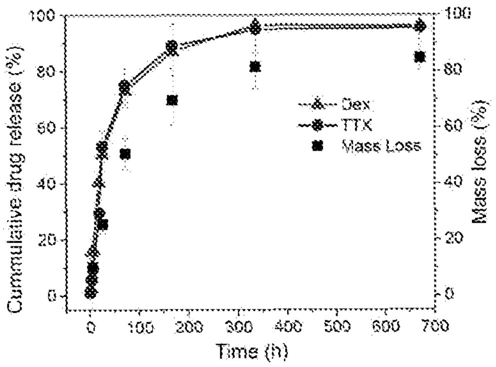


FIG. 6B

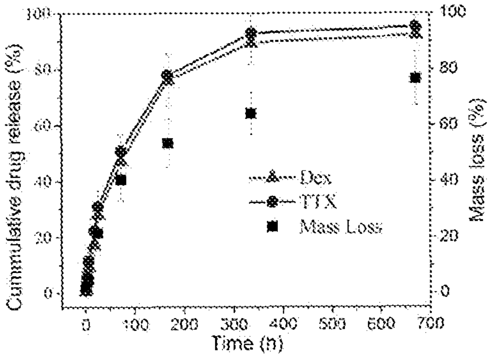


FIG. 6C

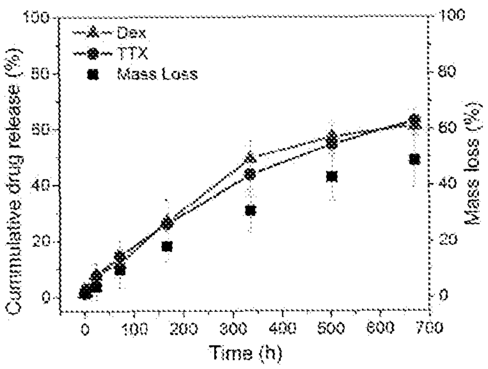


FIG. 6D

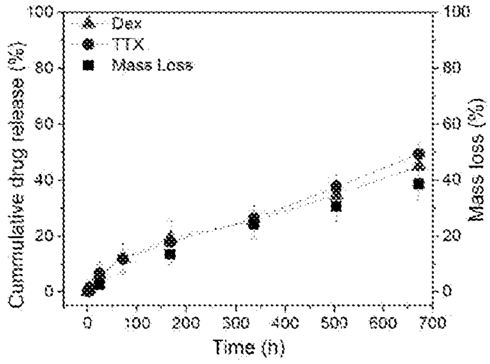


FIG. 6E

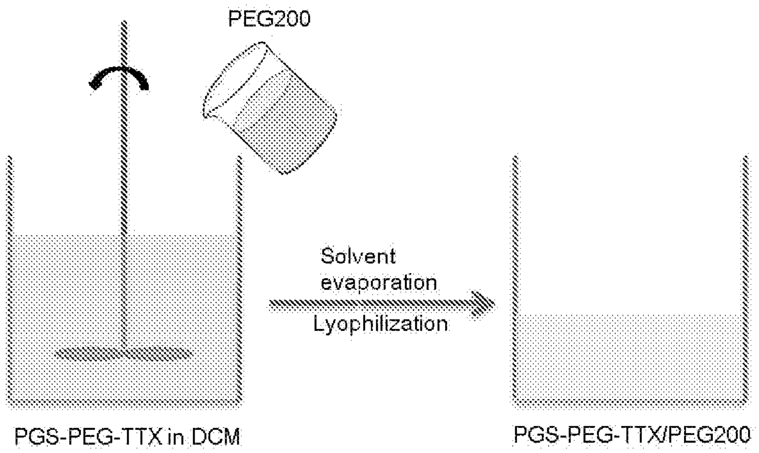


FIG. 7

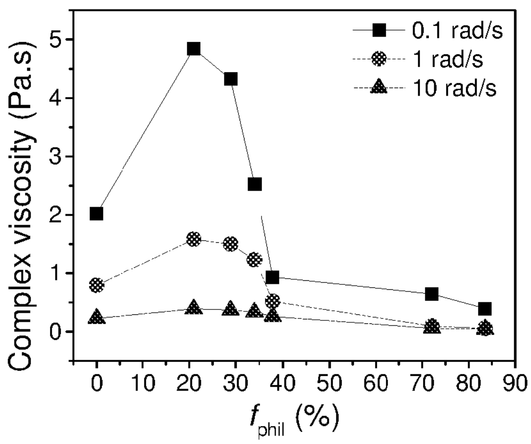


FIG. 8A

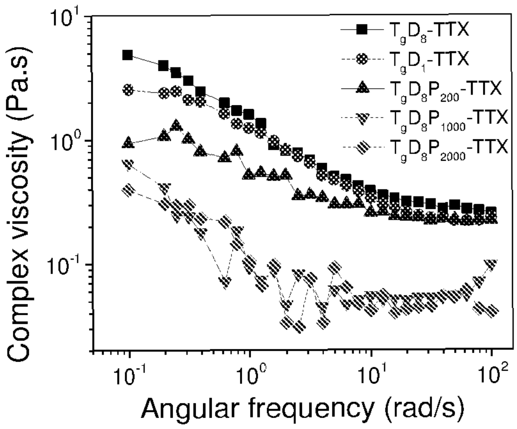


FIG. 8B

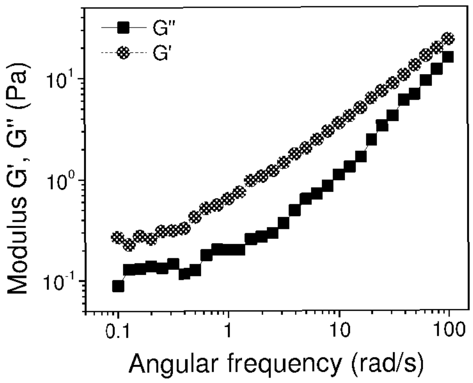


FIG. 8C

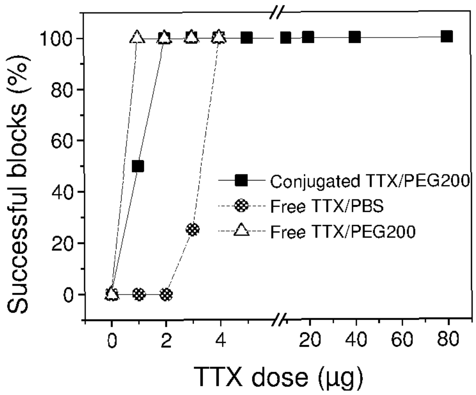


FIG. 9A

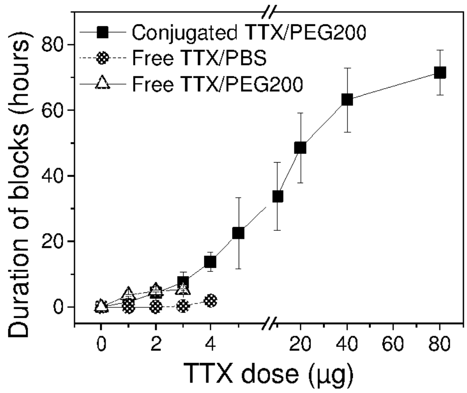


FIG. 9B

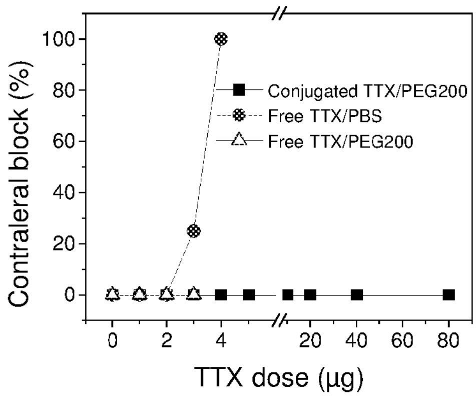


FIG. 9C

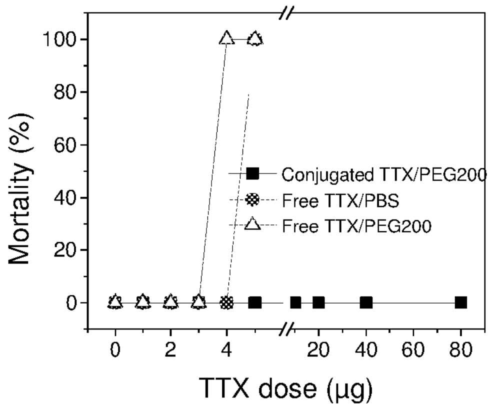


FIG. 9D

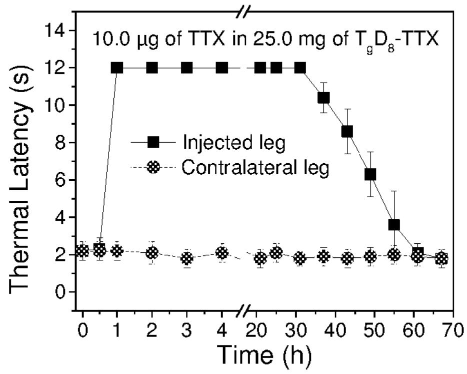


FIG. 10A

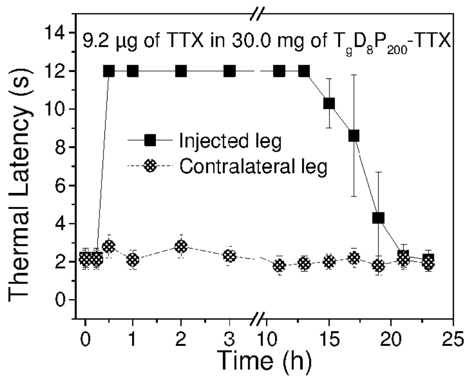


FIG. 10B

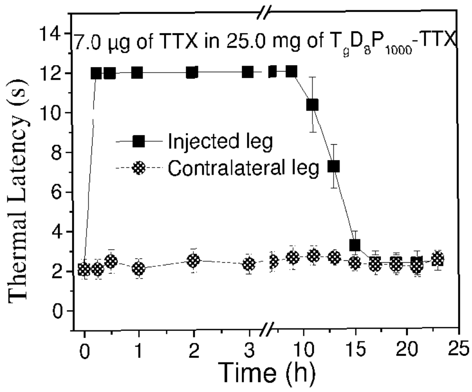


FIG. 10C

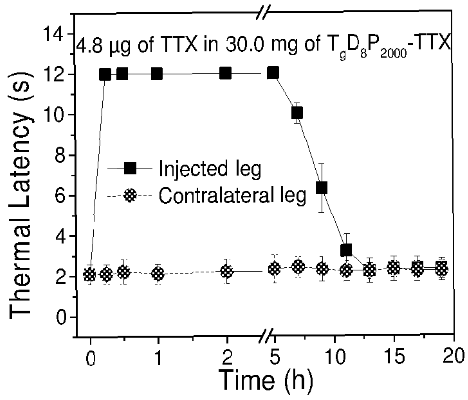


FIG. 10D

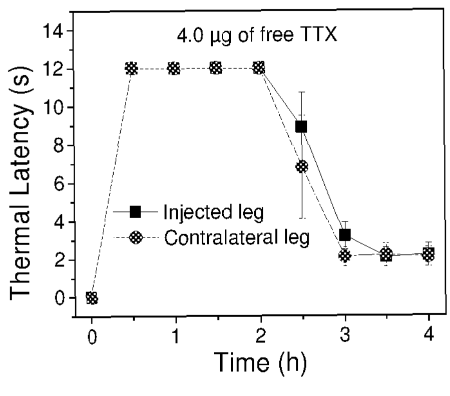


FIG. 10E

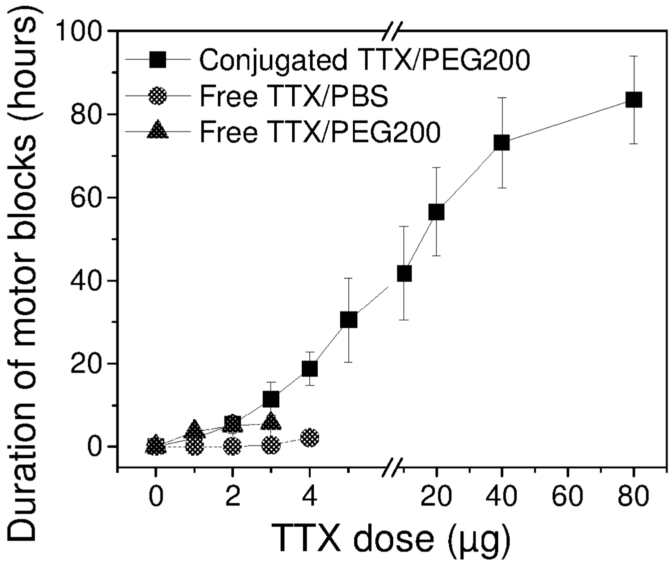


**FIG. 11**

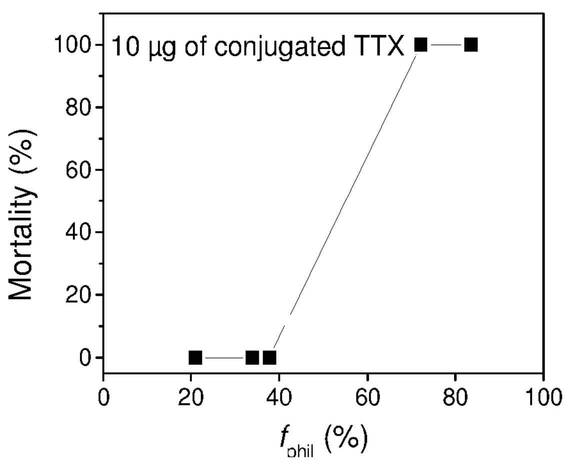


**FIG. 12A**

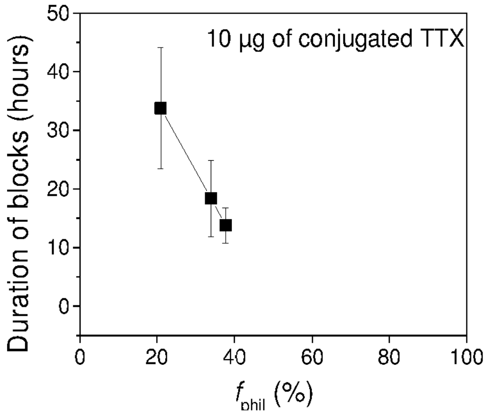


FIG. 12B

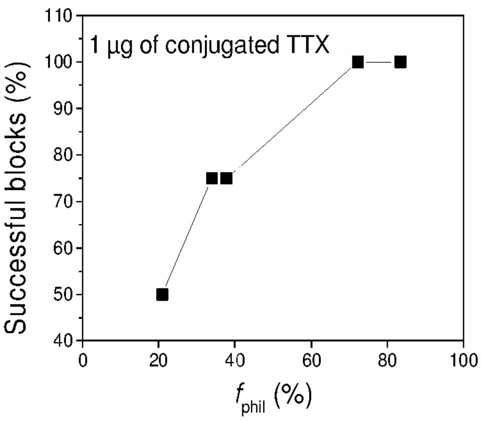


FIG. 12C

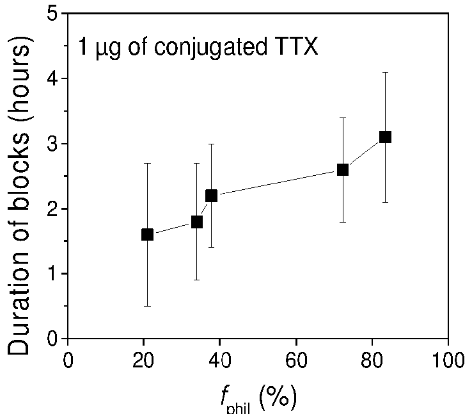


FIG. 12D

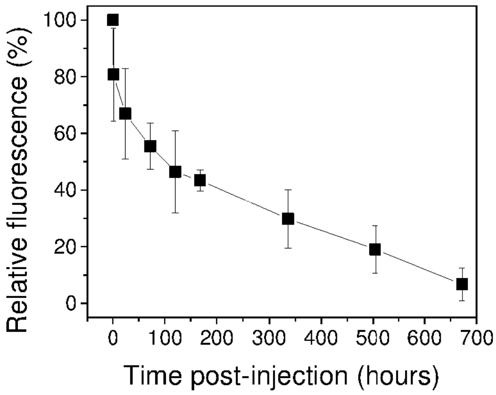
Relative fluorescence (%)
100
80
60
40
20
0
0
100
200
300
400
500
600
700
Time post-injection (hours)

**FIG. 13**

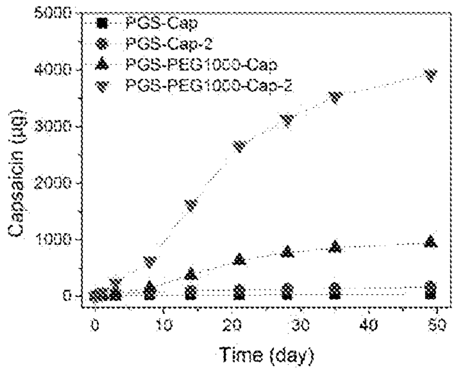
PGS-Cap
PGS-Cap-2
PGS-PEG1000-Cap
PGS-PEG1000-Cap-2
Capsaicin (μg)
5000
4000
3000
2000
1000
0
0
10
20
30
40
50
Time (day)

**FIG. 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4001413 A


### Non-patent literature cited in the description


- **PADERA et al.** *Anesthesiology*, 2008, vol. 108, 921-928
- **KOHANE et al.** *Pain*, 2003, vol. 104, 415-421, http://dx.doi.org/10.1016/S0304-3959(03)00049-6
- **KOHANE et al.** *Journal of Biomedical Materials Research*, 2002, vol. 59, 450-459
- **LAHAYE et al.** *Anesthesiology*, 2015, vol. 123, 741-742
- **KOHANE et al.** *Regional Anesthesia and Pain Medicine*, 2000, vol. 25, 52-59, http://dx.doi.org/10.1016/S1098-7339(00)80011-5
- **PERE et al.** *Regional Anesthesia and Pain Medicine*, 1993, vol. 18, 304-307
- **LOBO et al.** *Anesthesiology*, 2015, vol. 123, 873-885
- **RWEI et al.** *Proceedings of the National Academy of Sciences*, 2015, vol. 112, 15719-15724
- **SHANKARAPPA et al.** *Proceedings of the National Academy of Sciences*, 2012, vol. 109, 17555-17560
- **EPSTEIN-BARASH, H. et al.** *Proceedings of the National Academy of Sciences*, 2009, vol. 106, 7125-7130
- **ZHAN, C et al.** *Nano Letters*, 2016, vol. 16, 177-181
- *Pharmacological Reviews*, vol. 18 (2), 997-1049
- **KOHANE et al.** *Anesthesiology*, 1998, 119-131
- **OHYABU et al.** *J Am Chem Soc.*, 23 July 2003, vol. 125 (29), 8798-805
- **NISHIKAWA et al.** *Angew. Chem. Int. Ed*, 2004, vol. 43, 4782
- **CHAU** ; **CIUFOLINI**. *Mar Drugs*, 2011, vol. 9 (10), 2046-2074
- **KAO**. *C.Y., Pharm. Rev.*, 1966, vol. 18, 997-1049
- **RODRIGUEZ-NAVARRO et al.** *Anesthesiology*, 2007, vol. 106, 339-45
- **RODRIGUEZ-NAVARRO et al.** *, Neurotox. Res.*, 2009, vol. 16, 408-15
- **RODRIGUEZ-NAVARRO et al.** *, Reg. Anesth. Pain Med.*, 2011, vol. 36, 103-9
- **RODRIGUEZ-NAVARRO et al.** *Neurotox. Res.*, 2009, vol. 16, 408-15
- **LAGOS**. *N. Biol. Res.*, 1998, vol. 31, 375-386
- **LAGOS et al.** *TOXICON*, 1999, vol. 37, 1359-1373
- **PEREIRA et al.** *, TOXICON*, 2000, vol. 38, 1689-1702
- **KISHI et al.** *J. Am. Chem. Soc.*, 1977, vol. 98, 2818
- **JACOBI et al.** *J. Am. Chem. Soc.*, 1984, vol. 106 (19), 5594-5598
- **FLEMING et al.** *J. Am. Chem. Soc.*, 2006, 3926
- **KOHANE et al.** *Reg. Anesth. Pain Med.*, 2000, vol. 25, 52-9
- **RAI et al.** *Progress in Polymer Science V.*, 2012, vol. 37 (8), 1051-1078
- **JIA, Y. et al.** *Polymer Chemistry*, 2016, vol. 7, 2553-2564
- **RAI et al.** *Progress in Polymer Science*, 2012, vol. 37, 1051-1078
- **WANG et al.** *Journal of Biomedical Materials Research Part A*, 2003, vol. 66A, 192-197
- **LOH et al.** *Journal of Materials Chemistry B*, 2015, vol. 3, 7641-7652
- **WANG et al.** *Nat Biotech*, 2002, vol. 20, 602-606
- **MASTROPIETRO et al.** *J Dev Drugs*, 2013, vol. 2, 108
- **DEZANI et al.** *Brazilian Journal of Pharmaceutical Sciences*, 2013, vol. 49, 853-863
- **HIRATA, Y.** *Pure and Applied Chemistry*, 1978, vol. 50, 979
- **GEWERT, B.** ; **PLASSMANN, M. M.** ; **MACLEOD, M.** Pathways for degradation of plastic polymers floating in the marine environment.. *Environmental Science: Processes & Impacts*, 2015, vol. 17, 1513-1521
- **CHAFFIN, K. A.** ; **CHEN, X.** ; **MCNAMARA, L.** ; **BATES, F. S.** ; **HILLMYER, M. A.** Polyether Urethane Hydrolytic Stability after Exposure to Deoxygenated Water.. *Macromolecules*, 2014, vol. 47, 5220-5226

- **MASTROPIETRO, D.** ; **NIMROOZI, R.** ; **OMIDIAN, H.** Rheology in pharmaceutical formulations-A perspective.. *J Dev Drugs*, 2013, vol. 2, 108
- **RWEI, A. Y. et al.** Repeatable and adjustable on-demand sciatic nerve block with phototriggerable liposomes.. *Proceedings of the National Academy of Sciences*, 2015, vol. 112, 15719-15724
- **LAHAYE, L. A.** ; **BUTTERWORTH, I. V. J. F.** Site-1 Sodium Channel Blockers as Local AnestheticsWill Neosaxitoxin Supplant the Need for Continuous Nerve Blocks?. *Anesthesiology*, 2015, vol. 123, 741-742
- **RAI, R.** ; **TALLAWI, M.** ; **GRIGORE, A.** ; **BOCCACCINI, A. R.** Synthesis, properties and biomedical applications of poly(glycerol sebacate) (PGS): A review.. *Progress in Polymer Science*, 2012, vol. 37, 1051-1078
- **WANG, Y.** ; **KIM, Y. M.** ; **LANGER, R.** In vivo degradation characteristics of poly(glycerol sebacate). *Journal of Biomedical Materials Research Part A*, 2003, vol. 66A, 192-197
- **MCALVIN, J. B.** ; **REZNOR, G.** ; **SHANKARAPPA, S. A.** ; **STEFANESCU, C. F.** ; **KOHANE, D. S.** Local Toxicity from Local Anesthetic Polymeric Microparticles.. *Anesthesia and analgesia*, 2013, vol. 116, 794-803
- **F PADERA, R.** ; **TSE, J.** ; **BELLAS, E** ; **S KOHANE, D.** *Tetrodotoxin for prolonged local anesthesia with minimal myotoxicity.*, 2006, vol. 34
- **D'SOUZA, A. A.** ; **SHEGOKAR, R.** Polyethylene glycol (PEG): a versatile polymer for pharmaceutical applications.. *Expert Opinion on Drug Delivery*, 2016, vol. 13, 1257-1275